# EUROPEAN PATENT SPECIFICATION

(11) **EP 3 628 684 B1**
(45) Date of publication and mention of the grant of the patent: **18.09.2024**
(21) Application number: 19208285.7
(22) Date of filing: 08.06.2015
(51) Int. Cl.: C07K 14/74, C12N 15/10, G01N 33/543, G01N 33/569, C07K 19/00

(54) **DETERMINING ANTIGEN RECOGNITION THROUGH BARCODING OF MHC MULTIMERS**
BESTIMMUNG VON ANTIGENERKENNUNG DURCH BARCODING VON MHC-MULTIMEREN
DÉTERMINATION DE RECONNAISSANCE D'ANTIGÈNE PAR L'INTERMÉDIAIRE D'UN MARQUAGE PAR CODE-BARRES DE MULTIMÈRES DU CMH

(30) Priority: 06.06.2014 DK PA201470340
(43) Date of publication of application: 01.04.2020
(62) Divisional of application: 15739508.8
(73) Proprietor: Herlev Hospital, 2730 Herlev (DK); Immudex ApS, 2830 Virum (DK)
(72) Inventor: HADRUP, Sine Reker, 2830 Virum (DK); PEDERSEN, Henrik, 2880 Bagsværd (DK); JAKOBSEN, Søren, 2900 Hellerup (DK); BENTZEN, Amalie Kai, 1852 Frederiksberg C (DK)
(74) Representative: Høiberg P/S

(56) References cited:
- WO-A1-2013/137737
- WO-A2-2009/077173
- WO-A2-2009/126828
- WO-A2-2012/094492
- SENECI P: "Encoding techniques for pool libraries of small organic molecules", JOURNAL OF RECEPTOR AND SIGNAL TRANSDUCTION RESEARCH, MARCEL DEKKER, NEW YORK, NY, US, vol. 21, no. 4, November 2001 (2001-11-01), pages 409 - 445, XP009086023, ISSN: 1079-9893, DOI: 10.1081/RRS-100107925
- LEONID V. BYSTRYKH: "Generalized DNA Barcode Design Based on Hamming Codes", PLOS ONE, vol. 7, no. 5, 17 May 2012 (2012-05-17), pages e36852, XP055560951, DOI: 10.1371/journal.pone.0036852

## Description

### Technical field

The present disclosure relates to antigen recognition through nucleic acid labelled MHC multimers.

### Background

The adaptive immune system is directed through specific interactions between immune cells and antigen-presenting cells (e.g. dendritic cells, B-cells, monocytes and macrophages) or target cells (e.g. virus infected cells, bacteria infected cells or cancer cells). In important field in immunology relates to the understanding of the molecular interaction between an immune cell and the target cell.

Specifically for T-lymphocytes (T-cells), this interaction is mediated through binding between the T-cell receptor (TCR) and the Major Histocompatibility Complex (MHC) class I or class II. The MHC molecules carries a peptide cargo, and this peptide in decisive for T-cell recognition. The understanding of T-cell recognition experienced a dramatic technological breakthrough when Atman et al. (1) in 1996 discovered that multimerization of single peptide-MHC molecules into tetramers would allow sufficient binding-strength (avidity) between the peptide-MHC molecules and the TCR to determine this interaction through a fluorescence label attached to the MHC-multimer. Such fluorescent-labelled MHC multimers (of both class I and class II molecules) are now widely used for determining the T-cell specificity. The MHC multimer associated fluorescence can be determined by e.g. flow cytometry or microscopy, or T-cells can be selected based on this fluorescence label through e.g. flow cytometry or bead-based sorting. However, a limitation to this approach relates to the number of different fluorescence labels available, as each fluorescence label serve as a specific signature for the peptide-MHC in question.

Thus, this strategy is poorly matching the enormous diversity in T-cell recognition. For the most predominant subset of T-cells (the αβ TCR T-cells), the number of possible distinct αβ TCRs has been estimated at ~10¹⁵ (2) although the number of distinct TCRs in an individual human is probably closer to 10⁷ (3). Therefore, much effort has attempted to expand the complexity of the T-cell determination, with the aim to enable detection of multiple different T-cell specificities in a single sample. A more recent invention relates to multiplex detection of antigen specific T-cells is the use of combinatorial encoded MHC multimers. This technique uses a combinatorial fluorescence labelling approach that allows for the detection of 28 different T-cell populations in a single sample when first published (4,5), but has later been extended through combination with novel instrumentation and heavy metal labels to allow detection of around 100 different T-cell populations in a single sample (6).

The requirement for new of technologies that allow a more comprehensive analysis of antigen-specific T-cell responses is underscored by the fact that several groups have tried to develop so-called MHC microarrays. In these systems, T-cell specificity is not encoded by fluorochromes, but is spatially encoded (7,8). In spite of their promise, MHC microarrays have not become widely adopted, and no documented examples for its value in the multiplexed measurement of T-cell responses, for instance epitope identification, are available.

WO 2009/126828 discloses multiplex sorting of cells and subsequent analysis of said sorted cells, including detection of T cells.

WO 2009/077173 discloses methods of preparing DNA-encoded chemical libraries.

WO 2012/094492 and WO 2009/077173 disclose methods to identify single compounds using a DNA barcode.

WO 2013/137737 and Seneci et al., 2001 ("Encoding techniques for pool libraries of small organic molecules", Journal of Receptor and signal transduction Research, vol. 21 no. 4 p. 409-445) disclose the use of PCR amplification to identify molecules tagged by a barcode flanked by conserved primers.

Kwong, Gabriel Agner ('2009') ("DNA Encoded Biotechnologies for Informative cancer Diagnostics", Dissertation (Ph.D.), California Institute of Technology) discloses detection of T cells using cellular barcoding including a step of cleaving barcodes from the capture agent before their amplification.

Considering the above, there remains a need for a high-throughput method in the art of detection, isolation and/or identification of specific antigen responsive cells, such as antigen specific T-cells.

Further, there remains a need in the art, considering the often limited amounts of sample available, for methods allowing detection, isolation and/or identification of multiple species of specific antigen responsive T-cells in a single sample.

### Summary

The present disclosure relates to the use of nucleic acid-barcodes for the determination and tracking of antigen specificity of immune cells.

In an aspect of the present disclosure a nucleic acid-barcode will serve as a specific label for a given peptide-MHC molecule that is multimerized to form a MHC multimer. The multimer can be composed of MHC class I, class II, CD1 or other MHC-like molecules. Thus, when the term MHC multimers is used below this includes all MHC-like molecules. The MHC multimer is formed through multimerization of peptide-MHC molecules via different backbones. The barcode will be co-attached to the multimer and serve as a specific label for a particular peptide-MHC complex. In this way up to 1000 to 10.000 (or potentially even more) different peptide-MHC multimers can be mixed, allow specific interaction with T-cells from blood or other biological specimens, wash-out unbound MHC-multimers and determine the sequence of the cell-bound DNA-barcodes. When selecting a cell population of interest, the sequence of barcodes present above background level, will provide a fingerprint for identification of the antigen responsive cells present in the given cell-population. The number of sequence-reads for each specific barcode will correlate with the frequency of specific T-cells, and the frequency can be estimated by comparing the frequency of reads to the input-frequency of T-cells. This strategy may expand our understanding of T-cell recognition.

The DNA-barcode serves as a specific labels for the antigen specific T-cells and can be used to determine the specificity of a T-cell after e.g. single-cell sorting, functional analyses or phenotypical assessments. In this way antigen specificity can be linked to both the T-cell receptor sequence (that can be revealed by single-cell sequencing methods) and functional and phenotypical characteristics of the antigen specific cells.

Furthermore, this strategy may allow for attachment of several different (sequence related) peptide-MHC multimers to a given T-cell - with the binding avidity of the given peptide-MHC multimer determining the relative contribution of each peptide-MHC multimer to the binding of cell-surface TCRs. By applying this feature it is possible to allow the determination of the fine-specificity/consensus recognition sequence of a given TCR by use of overlapping peptide libraries or alanine substitution peptide libraries. Such determination is not possible with current MHC multimer-based technologies.

Thus, one aspect of the disclosure relates to a multimeric major histocompatibility complex (MHC) comprising
- two or more MHC's linked by a backbone molecule; and
- At least one nucleic acid molecule linked to said backbone, wherein said nucleic acid molecule comprises a central stretch of nucleic acids (barcode region) designed to be amplified by e.g. PCR.

Another aspect of the present disclosure relates to a composition comprising a subset of multimeric major histocompatibility complexes (MHC's) according to the disclosure, wherein each set of MHC's has a different peptide decisive for T cell recognition and a unique "barcode" region in the DNA molecule.

Yet another aspect of the present disclosure is to provide a kit of parts comprising
- a composition according to the disclosure; and
- one or more sets of primers for amplifying the nucleic acid molecules.

Still another aspect of the present disclosure is to provide a method for detecting antigen responsive T cells in a sample comprising:
- providing one or more multimeric major histocompatibility complexes (MHC's) according to the disclosure or a composition according to the disclosure;
- contacting said multimeric MHC's with said sample; and
- removing unbound multimeric MHCs before amplification by washing and spinning; and
- detecting binding of the multimeric MHC's to said antigen responsive T cells, thereby detecting T cells responsive to an antigen present in a set of MHC's,
wherein said binding is detected by amplifying by PCR the barcode region of said nucleic acid molecule linked to the one or more cell-bound multimeric MHC's; and sequencing of said amplified barcode regions.

Further aspects relates to different uses.

### Brief description of the figures

Fig. 1 describes the generation of barcode labelled MHC multimers.
Fig. 2 describes the generation of a library of barcode labelled MHC multimers.
Fig. 3 describes the detection of antigen responsive cells in a single sample.
Fig. 4 describes the possibility of linking the antigen specificity (tracked by the barcode) to other properties.
Fig. 5 shows in a set of experimental data that the disclosure is experimentally feasible.
Fig. 6 onwards shows experimental data of examples 2 onwards.

The present disclosurewill now be described in more detail in the following.

### Detailed description

The invention is defined by the appended claims. Any embodiment not falling under the scope of the appended claims does not form part of the invention.

### Definitions

Prior to discussing the present disclosure in further details, the following terms and conventions will first be defined:
TCR: T-cell receptor
MHC: Major Histocompatibility Complex
Multimeric MHC: Multimeric Major Histocompatibility Complex

### Nucleic acid barcode

In the present context, a nucleic acid barcode is a unique oligo-nucleotide sequence ranging for 10 to more than 50 nucleotides. The barcode has shared amplification sequences in the 3' and 5' ends, and a unique sequence in the middle. This sequence can be revealed by sequencing and can serve as a specific barcode for a given molecule.

### Sequencing

In the present aspect it is understood that sequencing also relates to e.g. deep-sequencing or next-generation sequencing, in which the amplified barcodes (the PCR product) is sequenced a large number of repetitive time (number of total reads, e.g. 100.000s of reads). The number of reads for the individual barcode sequence will relate to their quantitative presence in the amplification product, which again represents their quantitative presence before amplification, since all DNA-barcodes have similar amplification properties. Thus, the number of reads for a specific barcode sequences compared to the total number of reads will correlate to the presence of antigen responsive cells in the test-sample.

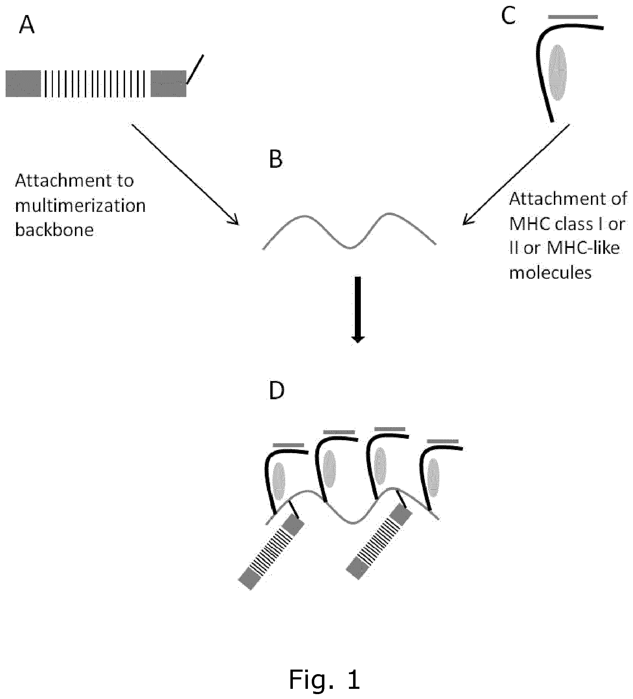

Referring now to the disclosure in more detail, Fig. 1 describes how peptide-MHC molecules, nucleic acid (DNA)-barcodes and (optional) fluorescent labels are assembled to form a library of MHC multimers each holding a DNA-barcode specific for the given peptide-MHC molecule involved. Fig 1A) the barcode is designed to have a unique sequences that can be determined through DNA sequencing. Also the barcode have shared amplification ends, enabling amplification of all DNA-barcodes simultaneously in a PCR reaction. DNA-barcodes are attached to the MHC-multimerization backbone (e.g. via a biotin linker binding to streptavidin on the multimer backbone). Fig 1B represents the multimer backbone. This may be any backbone that allow multimerization of macromolecules. The backbone may (optionally) hold a fluorescence label (illustrated by the asterisk) to track the total pool of MHC multimer binding cells irrespectively of the peptide-MHC multimer specificity. Fig 1C represents the peptide-MHC molecule of interest, carrying a specific peptide cargo (horizontal line). Fig. 1D represents the assembled peptide-MHC multimers carrying the DNA barcode.

### Multimeric major histocompatibility complex (MHC)

An aspect of the disclosure relates to a multimeric major histocompatibility complex (MHC) comprising
- two or more MHC's linked by a backbone molecule; and
- at least one nucleic acid molecule linked to said backbone, wherein said nucleic acid molecule comprises a central stretch of nucleic acids (barcode region) designed to be amplified by e.g. PCR.

Different types of backbones may be used. Thus, in an embodiment of the disclosure the backbone molecule is selected from the group consisting of polysaccharides, such as glucans such as dextran, a streptavidin or a streptavidin multimer. The skilled artisan may find other alternative backbones.

The MHC's may be coupled to the backbone by different means. Thus, in an embodiment of the disclosure the MHC's are coupled to the backbone through a streptavidin-biotin binding. Again other binding moieties may be used. The specific binding may use specific couplings points. In another embodiment of the disclosure the MHC's are linked to the backbone via the MHC heavy chain.

The MHC consists of different elements, which may partly be expressed and purified from cell systems (such as the MHC heavy chain and the Beta-2-microglobulin element). Alternatively, the elements may be chemically synthesized. The specific peptide is preferably chemically synthesized.

All three elements are required for the generation of a stable MHC (complex). Thus, in an embodiment of the disclosure the MHC is artificially assembled.

The multimeric MHC may comprise different numbers of MHC's. Thus, in yet an embodiment of the disclosure the multimeric major histocompatibility complex (MHC) is composed of at least four MHC's, such as at least eight, such as at least ten, 2-30, 2-20, such as 2-10 or such as 4-10 MHC's.

The nucleic acid component (preferably DNA) has a special structure. Thus, in an embodiment of the disclosure the at least one nucleic acid molecule is composed of at least a 5' first primer region, a central region (barcode region), and a 3' second primer region. In this way the central region (the barcode region) can be amplified by a primer set. The length of the nucleic acid molecule may also vary. Thus, in another embodiment of the disclosure the at least one nucleic acid molecule has a length in the range 20-100 nucleotides, such as 30-100, such as 30-80, such as 30-50 nucleotides. The coupling of the nucleic acid molecule to the backbone may also vary. Thus, in a further embodiment of the disclosure the at least one nucleic acid molecule is linked to said backbone via a streptavidin-biotin binding. Other coupling moieties may also be used.

In a further embodiment of the disclosure the at least one nucleic acid molecule comprises or consists of DNA, RNA, and/or artificial nucleotides such as PLA or LNA. Preferably DNA, but other nucleotides may be included to e.g. increase stability.

Different types of MHC's may form part of the multimer. Thus, in an embodiment of the disclosure the MHC is selected from the group consisting of class I MHC, a class II MHC, a CD1, or a MHC-like molecule. For MHC class I the presenting peptide is a 9-11mer peptide; for MHC class II, the presenting peptide is 12-18mer peptides. For alternative MHC-molecules it may be fragments from lipids or gluco-molecules which are presented.

It may also be advantageously if it was possible to determine the complete pool of bound multimers when incubated with a sample (of cells). Thus, in a preferred embodiment of the disclosure, the backbone further comprises one or more linked fluorescent labels. By having such coupling better quantification can be made. Similar the labelling may be used for cell sorting.

### Composition

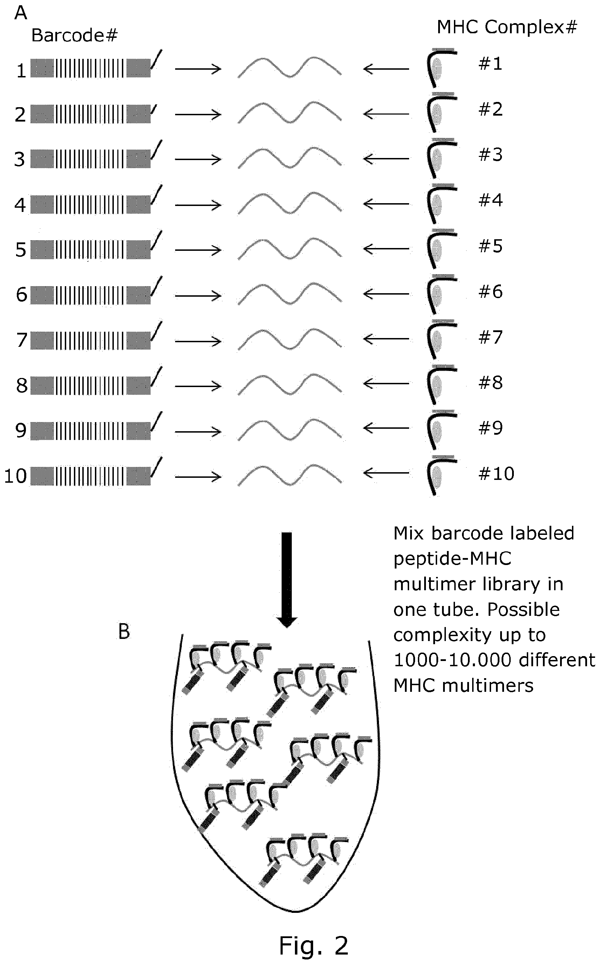

Fig 2 illustrates the generation of a full barcode library. Fig 2A, this library is composed of multiple, potentially more than 1000 different peptide-MHC multimers, each with a specific DNA-barcode. Such that barcode#1 codes for peptide-MHC complex#1, barcode#2 codes for peptide-MHC complex#2, barcode#3 codes for peptide-MHC complex#3, and so on until the possible mixture of thousands different specificities each with a specific barcode. Fig 2B represents the final reagent, which is a mixture of numerous different MHC-multimers each carrying a specific DNA barcode as a label for each peptide-MHC specificity.

As previously described a pool (library) of different sets of multimeric major histocompatibility complexes (MHC's) may be used to analyze an overall cell population for its specificity for peptides. Thus, another aspect of the disclosure relates to a composition comprising a subset of multimeric major histocompatibility complexes (MHC's) according to the disclosure, wherein each set of MHC's has a different peptide, decisive for T cell recognition and a unique "barcode" region in the DNA molecule. In the present context, it is to be understood that each specific multimeric major histocompatibility complex is present in the composition with a certain number and that there is subset of different multimeric major histocompatibility complexes present in the composition.

Preferably all specific region for each multimeric MHC can be determined with only a few primer sets, preferably only one primer set. Thus, in an embodiment of the disclosure the primer regions in the DNA molecule are identical for each set of MHC's. In this way only one primer set is required. In an alternative embodiment of the disclosure, the multimeric MHC's are grouped by different primer sets, thereby allowing multiplication of different sets of the cell-bound multimeric MHCs. In this way background noise may be limited, while also retrieving information of specific bindings. Thus, different primer set for different sets of cell-bound MHC's may be used.

The number of individual sets of multimeric MHC's may vary. Thus, in an embodiment of the disclosure the composition comprises at least 10 different sets of multimeric MHC's such as at least 100, such as at least 500, at least 1000, at least 5000, such as in the range 10-50000, such as 10-1000 or such as 50-500 sets of MHC's.

### Kit of parts

The composition of the disclosure may form part of a kit. Thus, yet an aspect of the disclosure relates to a kit of parts comprising
- a composition according to the disclosure; and
- one or more sets of primers for amplifying the DNA molecules.

### Method for detecting antigen responsive cells in a sample

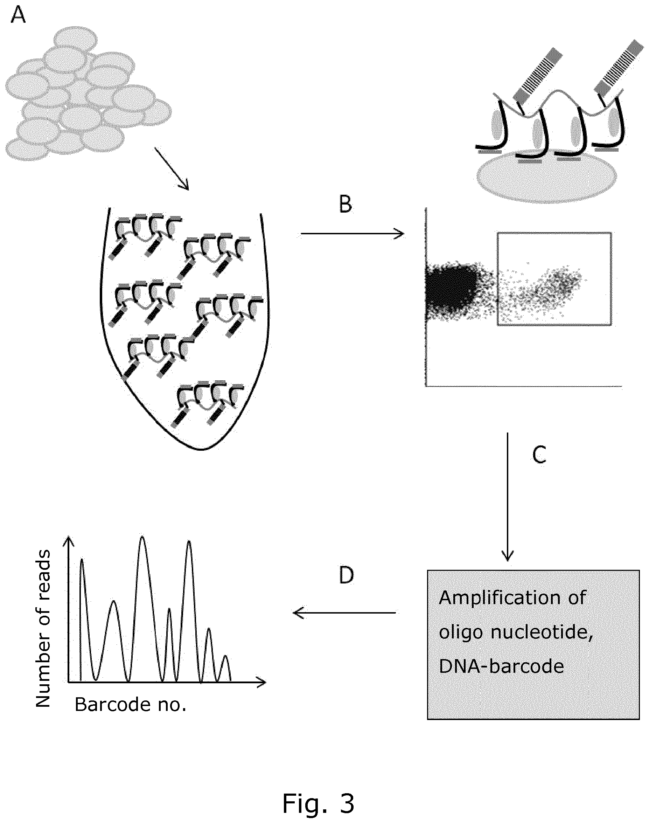

In Fig. 3 it is illustrated how this library can be used for staining of antigen responsive cells in a single sample. Fig 3A, cells in single cell suspension (may e.g., but not exclusive, originate from peripheral blood, tissue biopsies or other body fluids) are mixed with the peptide-library represented in fig 2B. Fig 3B, after staining, cells are sequentially washed and spun to remove residual MHC multimers that are not bound to a cellular surface. Specific cell populations, e.g. T-cells (CD8 or CD4 restricted), other immune cells or specifically MHC multimer binding T-cells may be sorted by flow cytometry or others means of cell sorting/selection. Fig 3C, the DNA-barcode oligonucleotide sequences bound to the cell population is amplified by PCR. Fig 2D, this amplification product is sequenced by deep sequencing (providing 10-100.000s of reads). The sequencing will reveal the specific barcode sequence of DNA barcodes attached to cells in the specimen after selection, as these will appear more frequent than sequences associated to the background of non-specific attachment of MHC multimers. The "signal-to-noise" is counteracted by the fact that any unspecific MHC multimer event will have a random association of 1/1000 different barcodes (dependent of the size of the library), making it even more sensitive than normal multimer staining.

Through analyses of barcode-sequence data, the antigen specificity of cells in the specimen can be determined. When DNA-barcode#1 is detected above background level of reads it means that peptide-MHC multimer#1 was preferentially bound to the selected cell type. Same goes for barcode no. 2, 3, 4, 5, ....etc. up to the potential combination of more than 1000 (nut not restricted to this particular number). When the number of input cells are known, e.g. when cell populations of interest is captured via a fluorescence signal also attached to the multimer by flow cytometry-based sorting or other means of capturing/sorting, the specific T-cell frequency can be calculated comparing the frequency of barcode-reads to the number of sorted T-cells.

Therefore, the multimeric MHC's and/or the compositions according to the disclosure may be used for different purposes. Thus, yet another aspect of the disclosure relates to a method for detecting antigen responsive T cells in a sample comprising:
- providing one or more multimeric major histocompatibility complexes (MHC's) or a composition according to the disclosure;
- contacting said multimeric MHC's with said sample; and
- removing unbound multimeric MHCs before amplification by washing and spinning; and
- detecting binding of the multimeric MHC's to said antigen responsive T cells, thereby detecting T cells responsive to an antigen present in a set of MHC's,
wherein said binding is detected by amplifying by PCR the barcode region of said nucleic acid molecule linked to the one or more cell-bound multimeric MHC's (through the backbone); and sequencing of said amplified barcode regions.

In an embodiment of the disclosure the method includes providing the (biological) sample.

As known to the skilled person, unbound molecules should be removed. Thus, in an embodiment of the disclosure unbound (multimeric) MHC's are removed before amplification, e.g. by washing and/or spinning e.g. followed by removing of the supernatant.

The type of sample may also vary. In an embodiment of the disclosure the sample is a biological sample. In an embodiment of the disclosure the sample is a blood sample, such as an peripheral blood sample, a blood derived sample, a tissue biopsy or another body fluid, such as spinal fluid, or saliva. The source of the sample may also vary. Thus, in a further embodiment of the disclosure said sample has been obtained from a mammal, such as a human, mouse, pigs, and/or horses.

It may also be advantageously to be able to sort the cells. Thus, in an embodiment of the disclosure the method further comprises cell sorting by e.g. flow cytometry such as FACS. This may e.g. be done if the backbone is equipped with a fluorescent marker. Thus, unbound cells may also be removed/sorted.

As also known to the skilled person, the measured values are preferably compared to a reference level. Thus, in an embodiment of the disclosure said binding detection includes comparing measured values to a reference level, e.g. a negative control and/or total level of response in the sample. In a further embodiment of the disclosure, said amplification is PCR such as QPCR.

As also previously mentioned the detection of the barcode includes sequencing of the amplified barcode regions. Thus, in an embodiment of the disclosure the detection of barcode regions includes sequencing of said barcode region, such as by deep sequencing or next generation sequencing.

### Use of a multimeric major histocompatibility complex

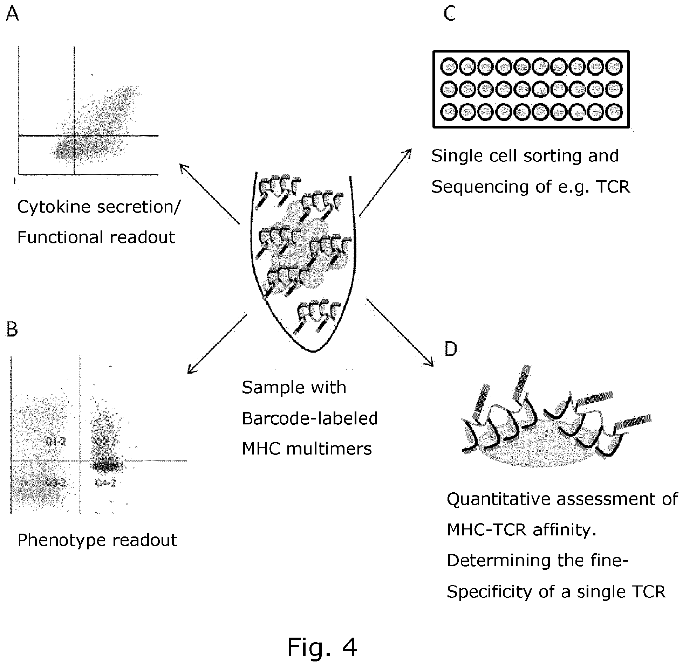

In Fig 4, it is illustrated how this technology can be used to link different properties to the antigen specificity of a cell population. Fig 4A. illustrates how cells after binding to a barcode labeled MHC multimer library may be exposed to a certain stimuli. Cell populations can be selected based on the functional response to this stimuli (e.g., but not exclusive, cytokine secretion, phosphorylation, calcium release or numerous other measures). After selecting the responsive or non-responsive population (following the steps of Fig 2), the DNA barcodes linked to the cell-bound multimeric MHCs can be sequenced to decode the antigen responsiveness, and thereby determining the antigen-specificities involved in a given response.

Fig 4B illustrates how cells can be selected based on phenotype, to link a certain set of phenotypic characteristics to the antigen-responsiveness.

Fig 4C represents the possibility for single-cell sorting of MHC-multimer binding cells based on the co-attached fluorescence label on the MHC multimer. Through single-cell sorting the antigen-specificity of the given cell can be determined on a single cell level through sequencing of the associated barcode label. This can be linked to the TCR that can also be sequenced on a single cell level, as recently described (10). Hereby, this disclosure will provide a link between the TCR sequence, or other single-cell properties and the antigen specificity, and may through the use of barcode labeled MHC multimer libraries enable definition of antigen-specific TCRs in a mixture of thousands different specificities.

Fig 4D illustrates the use of barcode labeled MHC multimer libraries for the quantitative assessment of MHC multimer binding to a given T-cell clone or TCR transduced/transfected cells. Since sequencing of the barcode label allow several different labels to be determined simultaneously on the same cell population, this strategy can be used to determine the avidity of a given TCR relative to a library of related peptide-MHC multimers. The relative contribution of the different DNA-barcode sequences in the final readout is determined based on the quantitative contribution of the TCR binding for each of the different peptide-MHC multimers in the library. Via titration based analyses it is possible to determine the quantitative binding properties of a TCR in relation to a large library of peptide-MHC multimers. All merged into a single sample. For this particular purpose the MHC multimer library may specifically hold related peptide sequences or alanine-substitution peptide libraries.

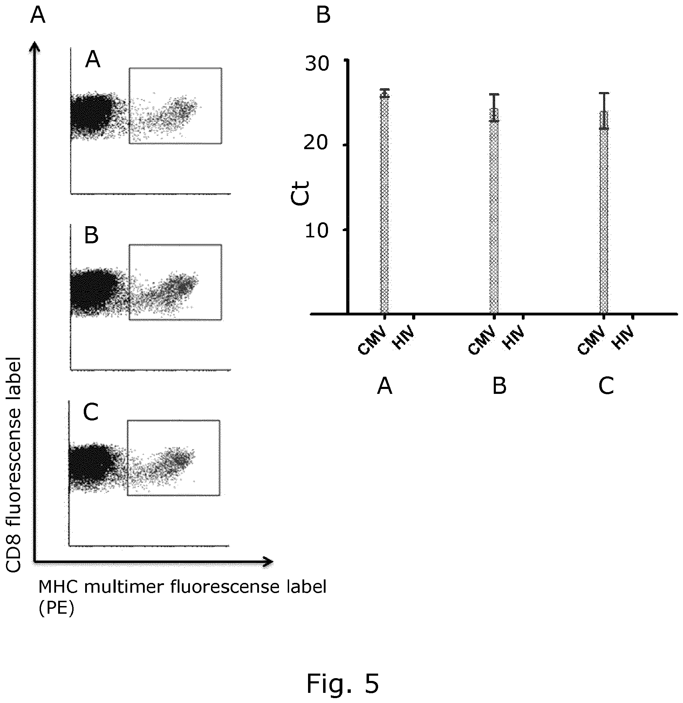

Fig 5 shows experimental data for the feasibility of attaching a DNA-barcode to a MHC multimer and amplify the specific sequences following T-cell staining. Fig 5A shows the staining of cytomegalovirus (CMV) specific T-cells in a peripheral blood samples. The specific CMV-derived peptide-MHC multimers was labeled with a barcode (barcode#1) and mixed with an irrelevant/non-specific peptide-MHC multimer labeled with barcode (barcode#2) and mixed with 998 other non-barcode labeled non-specific MHC multimers. Data here shows the feasibility for staining of CMV-specific T-cells in a mixture of 1000 other MHC multimers. Data is shown for three different staining protocols. Fig 5B shows the readout of the specific barcode sequences by quantative PCR. Barcode#1 (B#1) determining the CMV specific T-cell in detected for all three staining protocols, whereas the irrelevant/non-specific barcode signal, barcode#2 (B#2) is undetectable.

Overall, the multimeric MHC's or compositions comprising such sets of MHC's may find different uses. Thus, an aspect relates to the use of a multimeric major histocompatibility complex (MHC) or a composition according to the disclosure for the detecting of antigen responsive T cells in a sample.

Another aspect relates to the use of a multimeric major histocompatibility complex (MHC) or a composition according to the disclosure in the diagnosis of diseases or conditions, preferably cancer and/or infectious diseases.

A further aspect relates to the use of a multimeric major histocompatibility complex (MHC) or a composition according to the disclosure in the development of immune-therapeutics.

Yet a further aspect relates to the use of a multimeric major histocompatibility complex (MHC) or a composition according to the disclosure in the development of vaccines.

Another aspect relates to the use of a multimeric major histocompatibility complex (MHC) or a composition according to the disclosure for the identification of epitopes.

In sum, the advantages of the present disclosure include, without limitation, the possibility for detection of multiple (potentially, but exclusively, >1000) different antigen responsive T cells in a single sample. The technology can be used, but is not restricted, for T-cell epitope mapping, immune-recognition discovery, diagnostics tests and measuring immune reactivity after vaccination or immune-related therapies.

This level of complexity allow us to move from model antigens to determination of epitope-specific immune reactivity covering full organisms, viral genomes, cancer genomes, all vaccine components etc. It can be modified in a personalized fashion dependent of the individuals MHC expression and it can be used to follow immune related diseases, such as diabetes, rheumatoid arthritis or similar.

Biological materials are for instance analyzed to monitor naturally occurring immune responses, such as those that can occur upon infection or cancer. In addition, biological materials are analyzed for the effect of immunotherapeutics including vaccines on immune responses. Immunotherapeutics as used here is defined as active components in medical interventions that aim to enhance, suppress or modify immune responses, including vaccines, non-specific immune stimulants, immunosuppressives, cell-based immunotherapeutics and combinations thereof.

The disclosure can be used for, but is not restricted to, the development of diagnostic kits, where a fingerprint of immune response associated to the given disease can be determined in any biological specimen. Such diagnostic kits can be used to determining exposure to bacterial or viral infections or autoimmune diseases, e.g., but not exclusively related to tuberculosis, influenza and diabetes. Similar approach can be used for immune-therapeutics where immune-responsiveness may serve as a biomarker for therapeutic response. Analyses with a barcode labelled MHC multimer library allow for high-throughput assessment of large numbers of antigen responsive T cells in a single sample.

Furthermore, barcode labelled MHC-multimers can be used in combination with single-cell sorting and TCR sequencing, where the specificity of the TCR can be determined by the co-attached barcode. This will enable us to identify TCR specificity for potentially 1000+ different antigen responsive T-cells in parallel from the same sample, and match the TCR sequence to the antigen specificity. The future potential of this technology relates to the ability to predict antigen responsiveness based on the TCR sequence. This would be highly interesting as changes in TCR usage has been associated to immune therapy (11,12).

Further, there is a growing need for the identification of TCRs responsible for target-cell recognition (e.g., but not exclusive, in relation of cancer recognition). TCRs have been successfully used in the treatment of cancer (13), and this line of clinical initiatives will be further expanded in the future. The complexity of the barcode labeled MHC multimer libraries will allow for personalized selection of relevant TCRs in a given individual.

Due to the barcode-sequence readout, the barcode labeled MHC multimer technology allow for the interaction of several different peptide-MHC complexes on a single cell surface, while still maintaining a useful readout. When one T-cell binds multiple different peptide-MHC complexes in the library, there relative contribution to T-cell binding can be determined by the number of reads of the given sequences. Based on this feature it is possible to determine the fine-specificity/consensus sequences of a TCR. Each TCR can potential recognize large numbers of different peptide-MHC complexes, each with different affinity (14). The importance of such quantitative assessment has increased with clinical used of TCRs and lack of knowledge may have fatal consequences as recently exemplified in a clinical study where cross recognition of a sequence related peptide resulted in fatal heart failure in two cases (15,16). Thus, this particular feature for quantitative assessment of TCR binding of peptide-MHC molecules related to the present disclosure, can provide an efficient solution for pre-clinical testing of TCRs aimed for clinical use.

Also related to the above, this allows for determination of antigen responsiveness to libraries of overlapping or to very similar peptides. Something that is not possible with present multiplexing technologies, like the combinatorial encoding principle. This allows for mapping of immune reactivity e.g. to mutation variant of viruses, such as, but nor exclusive, HIV.

In broad embodiment, the present disclosure is the use of barcode labelled MHC multimers for high-throughput assessment of large numbers of antigen responsive T cells in a single sample, the coupling of antigen responsiveness to functional and phenotypical characteristic, to TCR specificity and to determine the quantitative binding of large peptide-MHC libraries to a given TCR.

While the foregoing written description of the disclosure enables one of ordinary skill to make and use what is considered presently to be the best mode thereof, those of ordinary skill will understand and appreciate the existence of variations, combinations, and equivalents of the specific embodiment of the disclosure, method, and examples herein.

Additional items of the disclosure:
*Item* 1: Use of barcode labelled MHC multimers for multiplex detection of different T-cell specificities in a single sample, enabling simultaneous detection of potentially more than 1000 different T-cell specificities where the specificity is revealed through sequencing of the barcode label.
*Item 2:* Use of barcode labelled MHC multimers in combination with single-cell sorting and TCR sequencing, where the specificity of the TCR can be determined by the co-attached barcode. This will enable identification of TCRs specific for a mixture of numerous (potentially, but not restricted to >1000) different peptide-MHC multimers, and match the TCR sequence to the antigen specificity.
*Item 3:* Use of barcode labelled MHC multimers for determining the affinity and binding motif of a given TCR. The barcode labelling strategy will allow for attachment of several different (sequence related) peptide-MHC multimers to a given T-cell - with the binding affinity determining the relative contribution by each peptide-MHC multimer. Thereby it is possible to map the fine-specificity/consensus recognition sequence of a given TCR by use of overlapping peptide libraries or e.g. alanine substitution libraries.
*Item 4:* Use of barcode labelled MHC multimers to map antigen responsiveness against sequence related/similar peptides in the same libraries, e.g. mutational changes in HIV infection. This has not been possible with previous MHC multimer based techniques.
*Item 5:* The use of barcode-labelled MHC multimers to couple any functional feature of a specific T-cell or pool of specific T-cells to the antigen (peptide-MHC) recognition. E.g. determine which T-cell specificities in a large pool secrete cytokines, releases Calcium or other functional measurement after a certain stimuli.

It should be noted that embodiments and features described in the context of one of the aspects of the present disclosure also apply to the other aspects of the disclosure.

### Examples

### EXAMPLE 1

Figure 5 shows results that act as proof-of-principle for the claimed disclosure. Fig 5A, Flow cytometry data of peripheral blood mononuclear cells (PBMCs) from healthy donors.

### Materials and methods

PBMCs were stained with CMV specific peptide-MHC multimers coupled to a specific nucleotide-barcode. In addition to CMV peptide-MHC reagents the cells were stained in the presence of negative control reagents i.e. HIV-peptide MHC multimers coupled to another specific barcode label and the additional negative control peptide-MHC reagents (p*) not holding a barcode - all multimers were additionally labeled with a PE-fluorescence label. The amounts of MHC multimers used for staining of PBMCs were equivalent to the required amount for staining of 1000 different peptide-MHC specificities i.e. 1x oligo-labeled CMV specific MHC multimers, 1x oligo-labeled HIV specific MHC multimers and 998x non-labeled p*MHC multimers, so as to give an impression whether background staining will interfere with the true positive signal. Prolonged washing steps were included (either 0 min (A), 30 min (B) or 60 min (C)) after removing the MHC multimers, and data from all experiments are shown. The PE-MHC-multimer positive cells were sorted by fluorescence activated cell sorting (FACS)
Fig 5B, Cross threshold (Ct) values from multiplex qPCR of the sorted PE-MHC-multimer positive cells. QPCR was used to assess the feasibility of detecting certain cell specificity through barcode-labeled peptide-MHC-multimers. Reagents associated with a positive control (CMV) barcode and a negative control (HIV) barcode were present during staining, but negative control (HIV) barcode-peptide-MHC multimers should be washed out.

Examples of nucleic acid sequences are:
DNA-barcode oligo for CMV MHC multimer attachment: 
5GAGATACGTTGACCTCGTTGAANNNNNNTCTATCCATTCCATCCAGCTCACTTAAGC
TCTTGGTTGCAT
DNA-barcode oligo for HIV MHC multimer attachment: 
5GAGATACGTTGACCTCGTTGAANNNNNNTCTATAGGTGTCTACTACCTCACTTAAGC
TCTTGGTTGCAT
 5= Biotin-TEG

### Results

Results shows Ct value only detectable to the CMV peptide-MHC multimer associated barcode, whereas the HIV-peptide MHC multimer associated barcode was not detected

### Conclusion

This experiment is a representative example of several similar experiment performed with other antigen specificities. Overall these data show that it is feasible to
1) stain with 1000 different MHC-multimers in a single sample while still maintain a specific signal,
2) attach a DNA-barcode to an MHC multimer,
3) amplify the DNA-barcode after cellular selection steps,
4) read the barcode with QPCR, using barcode specific probes,
5) obtain a specific signal corresponding to the antigen specific T cell population present in the sample, while non-specific MHC multimer barcodes are non-detectable.

Together these (and similar data available) provide proof of feasibility for the steps described in figure 1, 2, and 3.

### EXAMPLE 2

This example relates to
i) the stability of DNA oligonucleotides, used in one embodiment of the disclosure, in blood preparations, and
ii) an embodiment of the disclosure, in which certain tagged Dextramers (detection molecules in which the binding molecule is a number of peptide-MHC complexes, and the label is a DNA oligonucleotide) are enriched for. Allowing identification of the Dextramers with binding specificity for certain (subpopulations of) cells in the cell sample tested.

In i) it is shown that DNA oligos are stable during handling in PBMC's and in blood for a time that will allow staining, washing and isolation of T cells and subsequent amplification of DNA tags.

In ii) Show that a model system consisting of DNA-tagged Dextramers with MHC specificities for CMV, Flu and negative control peptide will locate to and can be captured/sorted with relevant T cell specificities and can be identified by PCR amplification and/or sequencing.

### A. Stability of single-stranded and double-stranded oligonucleotides in blood preparations

DNA tag oligo design. 69-nucleotide long, biotinylated TestOligo consisting of 5'primer region (22nt yellow)-random barcode region (6xN-nt)-kodon region (21 nt green/underlined)-3'primer region (20nt blue) were prepared:
'b' = Biotin-TEG 5' modification
'h' = HEG (terminal modifications)

| | |
|---|---|
| Forward-01 primer | GAGATACGTTGACCTCGTTG |
| Reverse-01 primer | ATGCAACCAAGAGCTTAAGT |

TestOligo-01 
bGAGATACGTTGACCTCGTTGAANNNNNNTCTATCCATTCCATCCAGCTCACTTAAGC
TCTTGGTTGCAT
TestOligo-02 
bhGAGATACGTTGACCTCGTTGAANNNNNNTCTATCCATTCCATCCAGCTCACTTAAG
CTCTTGGTTGCAT
TestOligo-03 
bhGAGATACGTTGACCTCGTTGAANNNNNNTCTATCCATTCCATCCAGCTCACTTAAG
CTCTTGGTTGCATh
TestOligo-04 
bhGAGATACGTTGACCTCGTTGAANNNNNNTCTTGAACTATGAATCGTCTCACTTAAG
CTCTTGGTTGCATh
TestOligo-05 
bhGAGATACGTTGACCTCGTTGAANNNNNNTCTATAGGTGTCTACTACCTCACTTAAG
CTCTTGGTTGCATh
TestOligo-06

bhGAGATACGTTGACCTCGTTGAANNNNNNTCTTTATTGGAGAGCACGCTCACTTAAG
CTCTTGGTTGCATh

Q-PCR probes for quantifying the amount of TestOligos 1-6:
+ = locked nucleic acid (LNA) modified RNA nucleotide
LNA-3
8 = FAM; 7 = BHQ-1-plus
   TCT[+A][+T][+C]A[+T][+T]CC[+A][+T][+C]CAGC
LNA-4
8 = FAM; 7 = BHQ-1-plus
   TCT[+T][+G][+A]AC[+T][+A]TG[+A][+A][+T]CGTC
LNA-5
9 = HEX; 7 = BHQ-1-plus
   TCT[+A][+T][+A]GG[+T][+G]TC[+T][+A][+C]TACC
LNA-6
2 = Cy5; 1 = BHQ-2-plus
   TCT[+T][+T][+A]TT[+G][+G]AG[+A][+G][+C]ACGC

The stability of oligo-tags by Q-PCR was analyzed under conditions relevant for T cell isolation:
The testOligos 1-6 were incubated in anticoagulated EDTA blood, and following incubation the amount of each of the testOligos was determined using Q-PCR using the abovementioned primers and probes. The oligo tags were quantified by QPCR with SYBR^{®} Green JumpStart^{™} Taq ReadyMix^{™} according to manufacturer's protocol in combination with any capillary QPCR instruments (e.g. Roche LightCycler or Agilent Mx3005P).

Because of the different termini of the testOligos 1-6, this also was a test of the stability of non-modified DNA oligo tag vs HEG modified 5' and HEG modified 5' and 3' (TestOligo-01, -02 and -03 respectively).

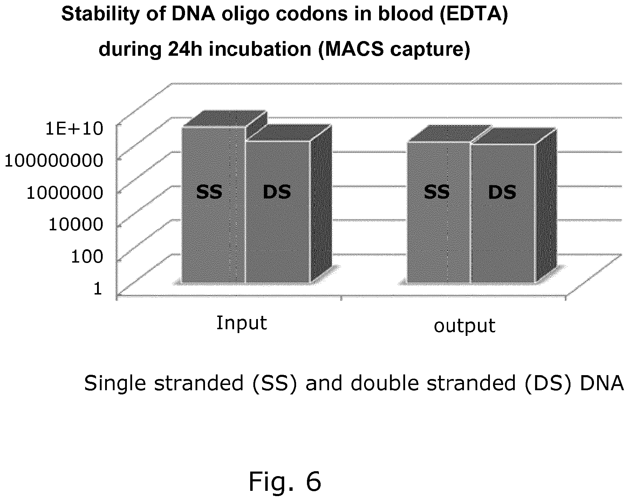

The results are shown in figure 6. It is concluded that the stability of the testOligos is appropriately high for all variants tested, to perform the disclosure.

### B. Generation and screening of a 3 member DNA tagged MHC Dextramer library for screening of antigen specific T cells in a lymphoid cell sample.

This experiment involves the generation of 3 DNA- tagged Dextramers, each with a unique specificity, as follows:
Dextramer 1: Flu (HLA-A*0201/GILGFVFTL/MP/lnfluenza)
Dextramer 2: CMV (HLA-A*0201/NLVPMVATV/pp65/CMV)
Dextramer 3: Negative (HLA-A*0201/ALIAPVHAV/Neg.Control).

Each of these Dextramers thus have a unique pMHC specificity (i.e. the three Dextramers have different binding molecules), and each Dextramer carries a unique label (DNA oligonucleotide) specific for that one pMHC specificity.

The library of DNA-tagged Dextramers are screened in a preparation of lymphoid cells such as anticoagulated EDTA blood or preparations of peripheral blood mononucleated cells (PBMC's). Those Dextramers that bind to cells of the cell sample will be relatively more enriched than those that do not bind.

Finally, the MHC/antigen specificity of the enriched Dextramers is revealed by identification of their DNA tags by Q-PCR with DNA tag-specific probes or by sequencing of the DNA tags.
1. Production of 3 different DNA tagged Dextramers with HLA-A*0201-peptide (pMHC) complexes.
   a. pMHC complexes are generated and attached to dextran, along with unique DNA tags identifying each of the individual pMHC complexes, as follows.
      i. Generation of DNA tagged Dextramers with Flu (HLA-A*0201/GILGFVFTL/MP/lnfluenza), CMV (HLA-A*0201/NLVPMVATV/pp65/CMV) and Negative (HLA-A*0201/ALIAPVHAV/Neg.Control).
         1. Dextramer stock is 160 nano molar (nM), TestOligo stock is diluted to 500 nM. Mix 10 micro liter (uL) 160 nM dextramer stock with 10 uL 500 nM TestOligo stock. Incubate 10 min at r.t. Mix with 1,5 ug pMHC complex of desired specificity. Adjust volume to 50 uL with a neutral pH buffer such as PBS or Tris pH 7,4, and store at 4 degrees Celsius. This will produce a DNA tagged Dextramer with approximately 3 oligo tags and 12 pMHC complexes, respectively, per Dextramer.
            a. Dex-Oligo-03 = Dextramer with TestOligo-03 and HLA-A*0201/NLVPMVATV/pp65/CMV.
            b. Dex-Oligo-04 = Dextramer with TestOligo-04 and HLA-A*0201/GILGFVFTL/MP/Influenza.
            c. Dex-Oligo-05 = Dextramer with TestOligo-05 and HLA-A*0201/ALIAPVHAV/Neg.Control.
2. Preparation of cell sample for screening for antigen-specific T cells.
   a. Appropriate cell samples for identification of antigen specific T cells are preparations of lymphoid cells such as preparations of peripheral blood mononucleated cells (PBMC's) or anticoagulated blood. Such preparations of cell samples are prepared by standard techniques known by a person having ordinary skill in the art.
   b. Transfer in the range of 1E7 lymphoid cells (from PBMC or EDTA anticoagulated blood) to a 12 x 75 mm polystyrene test tube.
   c. Add 2 ml PBS containing 5% fetal calf serum, pH 7.4. Centrifuge at 300 × g for 5 min. Remove supernatant and resuspend cells in a total volume of 2,5 ml PBS containing 5% fetal calf serum, pH 7.4.
3. Preparation and modification of library of DNA tagged Dextramers with three MHC/peptide specificities (from 1).
   a. Mix 5 ul 10 uM biotin with 10 ul each of Dex-Oligo-03, Dex-Oligo-04 and Dex-Oligo-05.
4. Mixing of preparations of lymphoid cells with a library of DNA tagged MHC Dextramers.
   a. Mix 1E7 lymphoid cells in 2,5 mL (from 2b) with 30 uL library of DNA tagged Dextramers (from 3a).
   b. Incubate 30 min at r.t.
   c. Centrifuge at 300 × g for 5 min. and remove the supernatant.
   d. Resuspend pellet in 2,5 ml PBS containing 5% fetal calf serum, pH 7.4. Centrifuge at 300 × g for 5 min. and remove the supernatant.
   e. Resuspend pellet in 2,5 ml PBS containing 5% fetal calf serum, pH 7.4
5. Capture of all CD8+ antigen specific T cells by magnet assisted cell sorting, performed according to Miltenyi Biotec catalog nr 130-090.878, Whole Blood CD8 MicroBead protocol.
   a. Ad 100 uL Whole Blood CD8 MicroBeads (Miltenyi Biotec catalog nr 130-090.878) to resuspended lymphoid cells from 4e. Mix and allow capture of CD8+ T cells for 15 min at r.t.
   b. Place Whole Blood Column in the magnetic field of a suitable MACS Separator. For details see the Whole Blood Column Kit data sheet.
   c. Prepare column by rinsing with 3 mL separation buffer (autoMACS Running Buffer or PBS containing 5% fetal calf serum, pH 7.4).
   d. Apply magnetically labeled cell suspension (4e) onto the prepared Whole Blood Column. Collect flow-through containing unlabeled cells.
   e. Wash Whole Blood Column with 3×3 mL separation buffer (autoMACS Running Buffer or PBS containing 5% fetal calf serum, pH 7.4).
   f. Remove Whole Blood Column from the separator and place it on a new collection tube.
   g. Capture CD8+ T cells by pipetting 5 mL Whole Blood Column Elution Buffer or PBS containing 5% fetal calf serum, pH 7.4 onto the Whole Blood Column. Immediately flush out the magnetically labeled cells by firmly pushing the plunger into the column.
   h. Centrifuge at 300 × g for 5 min. and remove the supernatant. Resuspend the collected CD8+ cells in 50 uL and store at minus 20 degrees Celsius for subsequent analysis.
6. Identification of Dextramers that bound significantly to antigen specific T cells of the lymphoid cell sample.
   a. Quantifying ratios of DNA oligo tags in input (3a) vs captured fraction (5h) by sequencing or alternatively quantifying by QPCR using the DNA tag specific probes LNA-3, LNA-4 and LNA-5 will reveal the relative abundance of antigen specific T cells in the lymphoid cell sample.
      i. Quantifying ratios of DNA oligo tags in input (3a) vs captured fraction (5h) by QPCR using the DNA tag specific probes LNA-3, LNA-4 and LNA-5.
         1. Make 25 uL QPCR reactions of
            a. input of library of DNA tagged Dextramers (3a)
            b. output of library of DNA tagged Dextramers (5h)
            c. Standard curves of 10 to 1E8 TestOligo-03, TestOligo-04 and TestOligo-05 respectively.
         2. Mix 12,5 uL JumpStart Taq ReadyMix (Sigma-Aldrich # D7440) with 0,125 uL 100 uM primer each of Forward-01 and Reverse-01, 0,625 ul 10 uM of either probe LNA-3, LNA-4 or LNA-5, 0,025 ul Reference dye (Sigma-Aldrich # R4526) and 12,5 uL of either input of library of DNA tagged Dextramers (3a), output of library of DNA tagged Dextramers (5h) or Standard curves of 10 to 1E8 TestOligo-03, TestOligo-04 and TestOligo-05 respectively.
         3. Run two step QPCR thermal profile Cycle 1 = 5 min at 95 degrees Celsius, Cycle 2-40 = 30 sec at 95 degrees Celsius and 1 min at 60 degrees Celsius.
         4. Estimate the relative abundance of T cells with antigen specificity against one of the three MHC Dextramers by plotting the QPC cycle time (Ct) values of the input of library of DNA tagged Dextramers (3a), the output of library of DNA tagged Dextramers (5h) in a plot of Ct values of the QPCR standard curve of TestOligo-03, TestOligo-04 and TestOligo-05 respectively.
      ii. Quantifying ratios of DNA oligo tags in input (3a) vs captured fraction (5h) by ultra-deep sequencing.
         1. Make 25 uL PCR reactions of
            a. input of library of DNA tagged Dextramers (3a)
            b. output of library of DNA tagged Dextramers (5h)
         2. Mix PCR reaction using any standard PCR master mix with 1,25 uL 10 uM primer each of Forward-01 and Reverse-01, and 12,5 uL of either input of library of DNA tagged Dextramers (3a) or output of library of DNA tagged Dextramers (5h). Top up to 25 uL with pure water. For example use 2x PCR Master Mix from Promega containing Taq DNA polymerase, dNTPs, MgCl2 and reaction buffers.
         3. Ultra Deep Sequencing of the above PCR product can be provided by a number of commercial suppliers such as for example Eurofins Genomics, GATC Biotech or Beckman Coulter Genomics using well established Next Generation Sequensing technologies such as Roche 454, Ion Torrent, the Illumina technology or any other high throughput sequencing technique for PCR amplicon sequencing.
         4. PCR amplicon analysis of the relative abundances of the input of library of DNA tagged Dextramers (3a), the output of library of DNA tagged Dextramers (5h) will reveal the relative abundance of T cells with antigen specificity against one of the three MHC Dextramers.
7. Predicted results and comments
   a. It is expected that the relative abundance and ratios of DNA oligo tags in input of a library of DNA tagged Dextramers (3a) as estimated by QPCR or sequencing is primarily affected by three parameters namely i) the ratio in which the DNA oligo tags were supplied during the generation of the DNA tagged Dextramers (1.a.i.1), ii) how the library input was mixed (3a) and iii) how efficiently the individual DNA oligo tags are amplified in the PCR reactions.
      i. In an example, the relative ratios of DNA oligo tags in input of a library of DNA tagged Dextramers as generated in 3a and as measured by QPCR or sequencing would be between 1 to 10 fold of each other.
   b. It is expected that the relative abundance and ratios of DNA oligo tags in the output of library of DNA tagged Dextramers (5h) as estimated by QPCR or sequencing, in addition to the three parameters mentioned in 7a, is primarily affected by three additional parameters namely i) the number of antigen specific T cells with specificity for one of the three MHC-peptide combinations ii) the affinity of the T cell receptor of the given T cell for the given MHC-peptide complex and finally iii) the efficiency of separating antigen-specific T cells and their associated DNA tagged MHC Dextramers from unbound DNA tagged MHC Dextramers by washing and cell capture.
      i. In an example, the relative ratios of DNA oligo tags in output of a library of DNA tagged Dextramers as generated in 5h and as measured by QPCR or sequencing would be more than 10 fold in favor of those DNA oligo tags coupled to an MHC Dextramer with an MHC-peptide complex for which antigen-specific T cells are present in the lymphoid cell sample.
         1. In a lymphoid cell sample from an influenza positive and CMV positive HLA-A0201 donor with antigen-specific T cells against HLA-A*0201/NLVPMVATV/pp65/CMV and HLA-A*0201/GILGFVFTL/MP/Influenza and no antigen-specific T cells against HLA-A*0201/ALIAPVHAV/Neg.Control it is expected that the relative ratios of TestOligo-03 (Dex-Oligo-03 = Dextramer with TestOligo-03 and HLA-A*0201/NLVPMVATV/pp65/CMV), TestOligo-04 (Dex-Oligo-04 = Dextramer with TestOligo-04 and HLA-A*0201/GILGFVFTL/MP/Influenza) and TestOligo-05 (Dex-Oligo-05 = Dextramer with TestOligo-05 and HLA-A*0201/ALIAPVHAV/Neg.Control) will be more than 10 fold in the favor of TestOligo-03 and TestOligo-04 over TestOligo-05. That is TestOligo-03 and TestOligo-04 is expected to be more than 10 fold more abundant or frequent than TestOligo-05 as measured by sequencing or QPCR of the output of library of DNA tagged Dextramers (5h) if they were supplied in equal amounts in the input of library of DNA tagged Dextramers (3a).

### EXAMPLE 3

This is an example where the Sample was blood from one CMV positive and HIV negative donor which was modified to generate Peripheral blood mononuclear cells (PBMCs). The Backbone was a dextran conjugate with streptavidin and fluorochrome (Dextramer backbone from Immudex).

The MHC molecules were peptide-MHC (pMHC) complexes displaying either CMV (positive antigen) or HIV (negative antigen) derived peptide-antigens. The MHC molecules were modified by biotinylation to provide a biotin capture-tag on the MHC molecule. The MHC molecule was purified by HPLC and quality controlled in terms of the formation of functional pMHC multimers for staining of a control T-cell population.

The oligonucleotide labels were synthetized by DNA Technology A/S (Denmark). The label was synthetically modified with a terminal biotin capture-tag. The labels were combined oligonucleotide label arising by annealing an A oligonucleotide (modified with biotin) to a partially complimentary B oligonucleotide label followed by enzymatic DNA polymerase extension of Oligo A and Oligo B to create a fully double stranded label. The MHC molecule was synthetized by attaching MHC molecules in the form of biotinylated pMHC and labels in the form of biotin-modified oligonucleotide onto a streptavidin-modified dextran backbone. The MHC molecule further contained a modification (5b) in the form of a fluorochrome. Two different MHC molecules were generated wherein the two individual MHC molecules containing different pMHC were encoded by corresponding individual oligonucleotide labels.

An amount of sample, PBMC's (1b) was incubated with an amount of mixed MHC molecules (5) under conditions (6c) that allowed binding of MHC molecules to T cells in the sample.

The cell-bound MHC molecules were separated from the non-cell bound MHC molecules (7) by first a few rounds of washing the PBMC's through centrifugation sedimentation of cells and resuspension in wash buffer followed by Fluorescence Activated Cell Sorting (FACS) of fluorochrome labeled cells. T cells that can efficiently bind MHC molecules will fluoresce because of the fluorochrome comprised within the MHC molecules; T cells that cannot bind MHC molecules will not fluoresce. FACS-sorting leads to enrichment of fluorescent cells, and hence, enrichment of the MHC molecules that bind T cells of the PBMC sample.

FACS isolated cells were subjected to quantitative PCR analysis of the oligonucleotide label associated with the MHC molecules bound to the isolated cells to reveal the identity of MHC molecules that bound to the T cells present in the sample.

This experiment thus reveal the presence of T cells in the blood expressing a T cell receptor that recognize/binds to peptide-MHC molecules comprised in the peptide-MHC multimeric library.
1. Sample preparation. The cell sample used in this experiment was obtained by preparing PBMC's from blood drawn from a donor that was CMV positive as well as HIV negative as determined by conventional MHC-multimer staining.
   a. Acquiring sample: Blood was obtained from the Danish Blood Bank
   b. Modifying sample: Peripheral blood mononuclear cells (PBMCs) were isolated from whole blood by density gradient centrifugation. The density gradient medium, Lymphoprep (Axis-Shield), which consists of carbohydrate polymers and a dense iodine compound, facilitate separation of the individual constituents of blood. Blood samples were diluted 1:1 in RPMI (RPMI 1640, GlutaMAX, 25mM Hepes; gibco-Life technologies) and carefully layered onto the Lymphoprep. After centrifugation, 30 min, 490g, PBMCs together with platelets were harvested from the middle layer of cells. The isolated cells, the buffy coat (BC), was washed twice in RPMI and cryopreserved at -150°C in fetal calf serum (FCS; gibco-Life technologies) containing 10% dimethyl sulfoxide (DMSO; Sigma-Aldrich). BC's used in this example are listed in table 6 together with their respective virus specificities. Their virus-specificities had been identified by conventional MHC multimer staining protocols.
2. BackboneBackbone preparation: The backbonebackbone is a dextran molecule, to which has been attached streptavidin and fluorochromes. The streptavidin serves as attachment sites for biotinylated oligonucleotides and biotinylated pMHC complexesMHC molecule. The fluorochrome allows separation of cells bound to MHC molecules from cells not bound to MHC molecules.
   a. In this example backbones were linear and branched dextran molecules of 1000-2000 KDa with covalently attached streptavidin (5-10 per backbone) and fluorochromes (2-20 per backbone) in the form of PE. Backbones are essentially Dextramer backbone as described by Immudex. In this example the backbones are also named SA conjugate.
3. MHC molecule preparation: The MHC molecules used in this example were two different class I MHC-peptide complexes. MHC heavy chains (HLA-A0201 and HLA-B0702) and B2M were expressed in E.coli as previously described (Hadrup et al. 2009) and each refolded with two peptide antigens. The individual specificities (peptide-MHC molecules, allele and peptide combination) were generated in the following way
   a. Synthesis: MHC molecules in this example was specific pMHC monomers that were produced from UV-exchange of selected HLA-I monomers carrying a UV-conditional 9-residue peptide-ligand (p*). When exposed to UV-light (366 nm) the conditional ligand will be cleaved and leave the binding groove empty. Due to the instability of empty MHC-I molecules, the complexes will quickly degrade if they are not rescued by replacement with another peptide that match that HLA-type. In this way specific pMHC monomers were produced by mixing excess of desired HLA ligands with p*MHC monomers. p*MHC monomers were refolded, biotinylated and purified as previously described (Hadrup et al. 2009).
      i. HIV derived peptide ILKEPVHGV from antigen HIV polymerase and CMV derived peptide TPRVTGGGAM from antigen pp65 TPR (Pepscan Presto, NL) were diluted in phosphate buffered saline (DPBS; Lonza) and mixed to final concentrations100µg/ml:200µM (HLA-A02: ILKEPVHGV and HLA-B07:TPRVTGGGAM). The mixtures were exposed to 366 nm UV light (UV cabinet; CAMAG) for one hour and optionally stored for up to 24 h at 4°C.
   b. Modification: No further modifications
   c. Purification: The panel of MHC molecules was moved to eppendorph tubes and centrifuged 5 min, 5000g, to sediment any MHC molecules not in solution, before being added to the cells.
4. Label preparation: In this example, two different oligonucleotides, of the same length but partially different sequence, were generated. Each of the oligonuclotides became attached to a specific pMHC, and thus encoded this specific pMHC. The oligonucleotides were biotinylated, allowing easy attachment to the dextran-streptavidin conjugate backbone.
   a. Synthesis: labels were DNA oligonucleotides which were purchased from DNA Technology (Denmark) and delivered as lyophilized powder. Stock dilutions of 100 µM label were made in nuclease free water and stored at - 20 °C.
      i. The label used was named 2OS label system and was developed to increase the complexity of a limited number of oligonucleotide sequences by a combinatorial label-generation strategy to produce multiple unique labels from a more confined number of label precursors. The strategy, referred to as 2OS, involved annealing and subsequent elongation of two partially complimentary oligonucleotide-sequences (an A oligo and a B oligo) that fostered a new unique oligonucleotide-sequences that was applied as a DNA oligonucleotide label. E.g. by combining 22 unique oligonucleotide-sequences (A label precursor) that are all partly complementary to 55 other unique oligonucleotide sequences (B label precursor) a combinatorial library of 1,210 different (Ax+By) labels could be produced (e.g. with 100 in table 9).
         1. Partly complementary A and B oligonucleotides were annealed to produce two combined A+B oligonucleotide labels (A1+B1 to produce A1B1 and A2+B2 to produce A2B2). A and a B oligos were mixed as stated in table 3, heated to 65 °C for 2 min and cooled slowly to <35°C in 15-30 min. The annealed A and B oligos were then elongated as stated in table 3. Components of the elongation reaction were mixed just before use. After mixing, the reaction was left 5 min at RT to allow elongation of the annealed oligonucleotides. The reagents used for annealing (left) and elongation (right) of partly complementary oligonucleotides is described in Table 3. Reagents marked in italic were from the the Sequenase Version 2.0 DNA Sequencing Kit (Affymetrix #70770).
   b. Modification: All labels were diluted to working concentrations (640nM) in nuclease free water with 0.1% Tween.
   c. Purification: No further purification of labels were performed.
5. MHC molecules preparation: The MHC molecules (pMHCs) and Labels (oligonucloetides) were attached to the backbone (backbone, dextran-streptavidine-fluorchrome conjugate), to form the MHC molecules, in a way so that a given pMHC is always attached to a given oligonucleotide.
   a. Synthesis: For preparation of MHC molecules the Backbone was labeled with the Label in the form of a biotinylated AxBx oligo prior to addition of pMHC.
      i. Creation of MHC molecules were performed by addition of label in two fold excess over backbone (2:1 label:backbone) and incubated at least 30 min, 4°C. Optionally the backbone were stored for up to 24 h at 4°C after coupling of the label. Prior to coupling MHC molecules, pMHC monomers, these were centrifuged 5 min, 3300g. SA conjugate (Dextramer backbone, Immudex) with conjugated streptavidin (SA) and fluorochrome (PE) were aliquoted into plates according to table 1. Avoiding the precipitate, MHC molecules were added to the aliquoted SA conjugate and incubated 30 min, RT. Following complex formation, D-biotin (Avidity Bio200) was added together with 0.02% NaN2 in PBS to the final concentration of pMHC monomer listed in table 1, and incubated at least 30 min or up to 24h at 4°C. Assembled MHC molecules were stored up to four weeks at 4°C. Two sets of two MHC molecules were generated. Each set with the two specificities individually labeled. The label was inverted between the two sets as described below.
         1. 1xCMV specific pMHCs coupled to 2OS-A1B1, 1xHIV specific pMHCs coupled to 2OS-A2B2
         2. 1xCMV specific pMHCs coupled to 2OS-A2B2, 1xHIV specific pMHCs coupled to 2OS-A1B1.
   b. Modification: No further modifications were performed
   c. Purification: MHC molecules were centrifuged 5 min, 3300g, to sediment any MHC molecules not in solution, before being added to the sample.
6. Incubation of sample and MHC molecules: The cell sample and the MHC molecules were mixed in one container, to allow the MHC molecules to bind the T cells that they recognize.
   a. Amount of sample: 1x10E6-2x10E6 cells in the form of BC's, were used.
   b. Amount of MHC molecule: According to table 1. 1 ug/ml calculated in relation to each MHC molecule (peptide-MHC molecule) was required per incubation
   c. Conditions: BCs were thawed in 10 ml, 37°C, RPMI with 10% fetal bovine serum (FBS), centrifuged 5 min, 490g, and washed twice in 10 ml RPMI with 10% FBS. All subsequent washing of cells refer to centrifugation 5 min, 490 g, with subsequent removal of supernatant. 2x10E6 cells were washed in 200ul barcode-buffer (PBS/0.5% BSA/2 mM EDTA/100 µg/ml herring DNA) and resuspended in this buffer to approximately 20µl per staining. Prior to incubation of cells with MHC molecules cells were incubated with 50 nM dasatinib, 30 min, 37°C (Lissina et al. 2009). MHC molecules were centrifuged for 5 min, 3300g, prior to addition to cells. 1 ug/ml 5 each MHC molecule (per pMHC) was required per incubation. After adding MHC molecules, the cells were incubated 15 min, 37°C. The antibody mixture listed in table 2 were added together with 0.1 µl near- IR-viability dye (Invitrogen L10119) that stains free amines. Antibody staining was essentially as for conventional MHC multimer staining. Cells were incubated 30 min, 4°C.
      Cells were then washed twice in 200 ul barcode buffer and incubated in 200 ul 1% paraformaldehyde in phosphate buffered saline (DPBS; Lonza) over night at 4°C.
7. Enrichment of MHC molecules with desired characteristics: In this Example, the MHC molecules were enriched by using flow cytometry, more specifically, Fluorescence-Activated-Cell-Sorting (FACS). The MHC molecules carry a fluorochrome. Hence, the cells that bind MHC molecules will fluoresce, and can, by applying a FACS sorter, be separated from the cells that do not bind MHC molecules and therefore do not fluoresce. As a result, the MHC molecules that bound to cells will be enriched for.
   a. Apply: Cells were sorted on a BD FACSAria, equipped with three lasers (488 nm blue, 633nm red and 405 violet). The flow cytometry data analyses was performed using the BD FACSDiva software version 6.1.2. The following gating strategy was applied. Lymphocytes were identified in a FSC/SSC plot. Additional gating on single cells (FSC-A/FSC-H), live cells (near-IR-viability dye negative), and CD4, CD14, CD16, CD19, CD40 negative (FITC)/CD8 positive cells (PerCP) were used to define the CD8 T cell population (table 2). The cells that were bound to at MHC molecule were defined within the PerCP positive population
   b. Wash: Cells were washed twice in barcode-buffer where after the cells were ready for flow cytometric acquisition. Optionally cells were fixed in 1% paraformaldehyde O.N., 4°C, and washed twice in barcode-buffer. Fixed cells were stored for up to a week at 4°C.
   c. Separate: Optionally cells were acquired up to one week after fixation in 1% paraformaldehyde. The multimer positive cells were sorted by FACS, as described in 7a, into tubes that had been pre-saturated for 2h-O.N. in 2% BSA and contained 200 µl barcode-buffer to increase the stability of the oligonucleotides that followed with the sorted cells. The sorted fluorochrome (PE) positive cells were centrifuged 5 min, 5000 g, to allow removal of all excess buffer. Cells were stored at -80 °C.
8. Identification of enriched MHC molecules: By identifying the Label (in this Example, the oligonucleotide label), the pMHCs that bound cells could be identified. Therefore, the oligonucleotides that were comprised within the MHC molecules that were recovered with the cells, were analyzed by quantitative PCR using Label-specific Q-PCR probes. This allowed the identification of pMHCs that bound cells of the cell sample.
   a. Labels derived from sorted cells were analyzed by QPCR according to table 4 QPCR was performed with the kit: Brilliant II QRT_PCR Low ROX Master Mix Kit (Agilent technologies, #600837). The thermal profile is listed in table 5. PCR was run on the thermal cycler: Mx3000P qPCR system (Agilent Technologies).

### Results and conclusions on Example 3

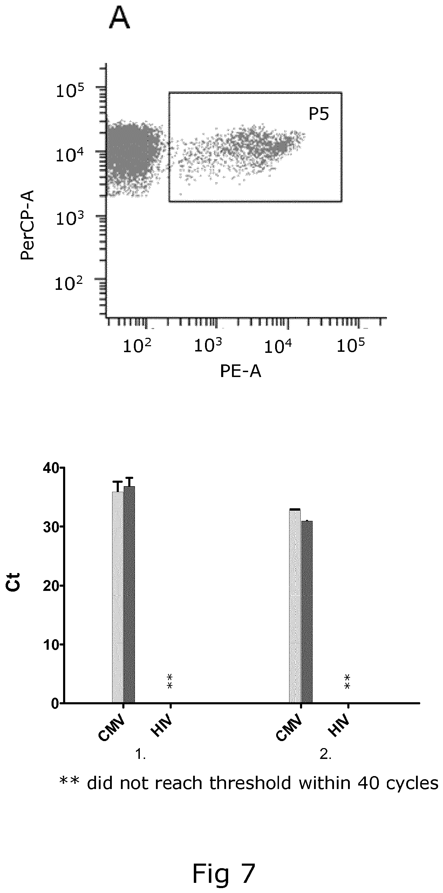

After sorting and qPCR the resultant Ct values confirmed that Labels were successfully recovered and enriched only when associated with the CMV epitope, while they were not detected when associated with the HIV epitope (figure 7).

Thus, it was verified that the 2OS labels were recovered after cellular interaction, sorting and qPCR only T cell recognizing the given pMHC molecule were present in the sample.

### Figure 7:

Detection of a B7 CMV pp65 TPR specificity amongst negative control barcoded pMHC dextramers. A unique 2OS barcode was associated with the positive control reagents in 1., while another unique 2OS barcode was associated with the positive control reagents in 2. The spare barcode in each experiment was associated with the HIV negative control reagent. A, Representative dot plot showing the PE positive population after staining with the CMV and HIV pMHC multimers carrying separate 2OS-barcodes. B, Ct values from multiplex qPCR of the sorted PE-pMHC-dextramer positive cells. Cells were stained with 1. and 2. respectively. Reagents associated with a positive control (CMV) 2OS barcode and a negative control (HIV) 2OS barcode were present during staining, but the negative control (HIV) barcoded pMHC dextramer was evidently washed out. The results obtained from two individual experiments are presented in separate bars. Approximately 200 cells were applied in each separate PCR. QPCR was run in duplicates and Ct values are shown as mean ±range of duplicates.

### Example 4

This is an example where the Sample (1) was blood from one CMV positive and HIV negative donor which was modified (1b) to generate Peripheral blood mononuclear cells (PBMCs).

The Backbone (2) was a dextran conjugate with streptavidin and fluorochrome (Dextramer backbone from Immudex).

The example is similar to example 1 except that a 1000 fold excess of MHC molecules with irrelevant MHC molecules but without label were included. The MHC molecules used (3) are peptide-MHC (pMHC) complexes displaying either CMV (positive antigen) or HIV (negative antigen) derived peptide-antigens or pMHC complexes displaying irrelevant peptide antigen. The MHC molecules were modified (3b) by biotinylation to provide a biotin capture-tag on the MHC molecule. The MHC molecules were purified (2c) by HPLC. The Labels (4) were oligonucleotides. The oligonucleotides were synthetized (4a) by DNA Technology A/S (Denmark). The labels were synthetically modified (4b) with a terminal biotin capture-tag.

The MHC molecule (5) was synthetized (5a) by attaching MHC molecules in the form of biotinylated pMHC and labels in the form of biotin-modified oligonucleotide onto a streptavidin-modified dextran backbone. The MHC molecule further contained a modification (5b) in the form of a fluorochrome. Three different MHC molecules were generated wherein the two of these individual MHC molecules containing CMV- and HIV-directed pMHC were encoded for by corresponding individual oligonucleotide labels. MHC molecules with irrelevant MHC molecules were not encoded for with oligonucleotide label. An amount of sample, PBMC's (1b) was incubated with an amount of mixed MHC molecules (5) in a ratio of 1:1 and in addition a 1000 fold of unlabeled p*MHC labeled backbone was included under conditions (6c) that allowed binding of MHC molecules to T cells in the sample.

The cell-bound MHC molecules were separated from the non-cell bound MHC molecules (7) by first a few rounds of washing the PBMC's through centrifugation sedimentation of cells and resuspension in wash buffer followed by Fluorescence Activated Cell Sorting (FACS) of fluorochrome labeled cells. T cells that can efficiently bind MHC molecules will fluoresce because of the fluorochrome comprised within the MHC molecules; T cells that cannot bind MHC molecules will not fluoresce. FACS-sorting leads to enrichment of fluorescent cells, and hence, enrichment of the MHC molecules that bind T cells of the PBMC sample.

FACS isolated cells were subjected to quantitative PCR analysis of the oligonucleotide label associated with the MHC molecules bound to the isolated cells to reveal the identity of MHC molecules that bound to the T cells present in the sample.

This experiment thus revealed the peptide-MHC specificity of the T cell receptors of the T cells present in the blood sample. It further revealed the feasibility of enriching for T cells specific for the CMV-antigen (positive) over the HIV-antigen (negative) and an excess of MHC molecule displaying irrelevant peptide antigens.
1. Sample preparation. The cell sample used in this experiment was obtained by preparing PBMC's from blood drawn from a donor that was CMV positive as well as HIV negative as determined by conventional MHC-multimer staining.
   a. Acquiring sample: Blood was obtained from the Danish Blood Bank
   b. Modifying sample: Peripheral blood mononuclear cells (PBMCs) were isolated from whole blood by density gradient centrifugation. The density gradient medium, Lymphoprep (Axis-Shield), which consists of carbohydrate polymers and a dense iodine compound, facilitate separation of the individual constituents of blood. Blood samples were diluted 1:1 in RPMI (RPMI 1640, GlutaMAX, 25mM Hepes; gibco-Life technologies) and carefully layered onto the Lymphoprep. After centrifugation, 30 min, 490g, PBMCs together with platelets were harvested from the middle layer of cells. The isolated cells, the buffy coat (BC), was washed twice in RPMI and cryopreserved at -150°C in fetal calf serum (FCS; gibco-Life technologies) containing 10% dimethyl sulfoxide (DMSO; Sigma-Aldrich). BC's used in this example are listed in table 6 together with their respective virus specificities. Their virus-specificities had been identified by conventional MHC multimer staining protocols.
2. Backbone preparation: The backbone used in this example is a dextran molecule, to which has been attached streptavidin and fluorochromes. The streptavidin serves as attachment sites for biotinylated oligonucleotides (Label) and biotinylated pMHC complexes (MHC molecules). The fluorochrome allows separation of cells bound to MHC molecules and cells not bound to MHC molecules.
   a. In this example backbones were linear and branched dextran molecules of 1000-2000 KDa with covalently attached streptavidin (5-10 per backbone) and fluorochromes (2-20 per backbone) in the form of PE. Backbones are essentially Dextramer backbone as described by Immudex. In this example the backbones are also named SA conjugate.
3. MHC molecules preparation: The MHC molecules used in this example were two different class I MHC-peptide complexes. MHC heavy chains (HLA-A02 and HLA-B07) and B2M were expressed in E.coli as previously described (Hadrup et al. 2009) and each refolded with two peptide antigens. The individual specificities (allele and epitope combination) were generated in the following way.
   a. Synthesis: A in experiment 1.
      i. As in experiment 1
   b. Modification: No further modifications
   c. Purification: as in experiment 1
4. Label preparation: In this experiment, two different oligonucleotides, of the same length but partially different sequence, were generated. Each of the oligonuclotides become attached to a specific pMHC, and thus encodes this specific pMHC. The oligonucleotides were biotinylated, allowing easy attachment to the dextran-streptavidine conjugate backbone.
   a. Synthesis: In this example the labels were DNA oligonucleotides which were purchased from DNA Technology (Denmark) and delivered as lyophilized powder. Stock dilutions of 100 µM label were made in nuclease free water and stored at -20 °C.
      i. As in experiment 1
      ii. Partly complementary A and B oligonucleotides were annealed to produce two combined A+B oligonucleotide labels (A1+B1 to produce A1B1 and A2+B2 to produce A2B2). A and a B oligos were mixed as stated in table 3, heated to 65 °C for 2 min and cooled slowly to <35°C in 15-30 min. The annealed A and B oligos were then elongated as stated in table 3. Components of the elongation reaction were mixed just before use. After mixing, the reaction was left 5 min at RT to allow elongation of the annealed oligonucleotides. The reagents used for annealing (left) and elongation (right) of partly complementary oligonucleotides is described in Table 3. Reagents marked in italic were from the the Sequenase Version 2.0 DNA Sequencing Kit (Affymetrix #70770).
   b. Modification: All labels were diluted to working concentrations (640nM) in nuclease free water with 0.1% Tween.
   c. Purification: No further purification of labels were performed.
5. MHC molecules preparation: The MHC molecules (pMHCs) and Labels (oligonucloetides) were attached to the backbone (dextran-streptavidine-fluorchrome conjugate), to form the MHC molecules, in a way so that a given pMHC is always attached to a given oligonucleotide.
   a. Synthesis: For preparation of MHC molecules the Backbone was labeled with the Label in the form of a biotinylated AxBx oligo prior to addition of pMHC.
      i. Creation of MHC molecules were performed by addition of label in two fold excess over backbone (2:1 label:backbone) and incubated 30 min, 4°C. Prior to coupling MHC molecules, pMHC monomers, these were centrifuged 5 min, 3300g. SA conjugate (Dextramer backbone, Immudex) with conjugated streptavidin (SA) and fluorochrome (PE) were aliquoted into tubes according to table 1. Avoiding the precipitate, MHC molecules were added to the aliquoted SA conjugate and incubated 30 min, RT. Following complex formation, D-biotin (Avidity Bio200) was added together with 0.02% NaN2 in PBS to the final concentration of pMHC monomer listed in table 1, and incubated 30 min, 4°C. Assembled MHC molecules were stored up to four weeks at 4°C. Two sets of two MHC molecules were generated. Each set with the two specificities individually labeled. The label was inverted between the two sets as described below.
         1. iv. 1xCMV specific pMHCs coupled to 2OS-A1B1, 1xHIV specific pMHCs coupled to 2OS-A2B2
         2. v. 1xCMV specific pMHCs coupled to 2OS-A2B2, 1xHIV specific pMHCs coupled to 2OS-A1B1.
   b. Modification: No further modifications were performed
   c. Purification: MHC molecules were centrifuged 5 min, 5000g, to sediment any MHC molecules not in solution, before being added to the sample.
6. Incubation of sample and MHC molecules: The cell sample and the MHC molecules were mixed in one container, to allow the MHC molecules to bind the T cells that they recognize.
   a. Amount of sample: 1x10E6-2x10E6 cells in the form of BC's, were used.
   b. Amount of MHC molecule: According to table 1. 5 ul of each MHC molecule was required per incubation (1 ug/ml in respect to pMHC)
   c. Conditions: BCs were thawed in 10 ml, 37°C, RPMI with 10% fetal bovine serum (FBS), centrifuged 5 min, 1500g, and washed twice in 10 ml RPMI with 10% FBS. All subsequent washing of cells refer to centrifugation 5 min, 490 g, with subsequent removal of supernatant. 1x10E6-2x10E6 cells were washed in barcode-buffer (PBS/0.5% BSA/2 mM EDTA/100 µg/ml herring DNA) and resuspended in this buffer to approximately 20µl per staining. Prior to incubation of cells with MHC molecules cells were incubated with 50nM dasatinib, 30 min, 37°C. MHC molecules were centrifuged for 5 min, 3300g, prior to addition to cells. 5 ul of each MHC molecule was required per incubation (1 ug/ml in respect to pMHC). After adding MHC molecules, the cells were incubated 15 min, 37°C. The antibody mixture listed in table 2 were added together with 0.1 µl near- IR-viability dye (Invitrogen L10119) that stains free amines. Antibody staining was essentially as for conventional MHC multimer staining . Cells were incubated 30 min, 4°C.
7. Enrichment of MHC molecules with desired characteristics: In this Example, the MHC molecules were enriched by using flow cytometry, more specifically, Fluorescence-Activated-Cell-Sorter (FACS). The MHC molecules carry a fluorochrome. Hence, the cells that bind MHC molecules will fluoresce, and can be separated from the cells that do not bind MHC molecules and therefore do not fluoresce, by a FACS sorter. As a result, the MHC molecules that bound to cells will be enriched for.
   a. Apply: Two different flow cytometers were used for acquisition. A BD FACSCanto II equipped with three lasers (488 nm blue, 633nm red and 405 violet) and a BD LSR II cytometer equipped with five lasers. Only four lasers on the LSR II were used throughout this study (488 nm blue laser, 640 nm red laser, 355 nm UV laser and 405 nm violet laser). Additionally cells were sorted on BD FACSAria and FACSAria II, equipped with three lasers (488 nm blue, 633nm red and 405 violet). All flow cytometry data analyses used the BD FACSDiva software version 6.1.2. The following gating strategy was used. All initial gatings of CD8 positive cells were performed alike. Lymphocytes were identified in a FSC/SSC plot. Additional gating on single cells (FSC-A/FSC-H), live cells (near-IR-viability dye negative), and dump-channel negative/CD8 positive cells (FITC/PerCP) were used to define the CD8 T cell population.
   b. Wash: Cells were washed twice in barcode-buffer where after the cells were ready for flow cytometric acquisition. Optionally cells were fixed in 1% paraformaldehyde O.N., 4°C, and washed twice in FACS buffer or barcode-buffer. Fixed cells were stored for up to a week at 4°C.
   c. Separate: Optionally cells were acquired up to one week after fixation in 1% paraformaldehyde. The multimer positive cells were sorted by FACS, as described in 7a, into tubes that had been pre-saturated for 2h-O.N. in 2% BSA and contained 200 µl barcode-buffer to increase the stability of the oligonucleotides that followed with the sorted cells. The sorted fluorochrome (PE) positive cells were centrifuged 5 min, 5000 g, to allow removal of all excess buffer. Cells were stored at -80 °C.
8. Identification of enriched MHC molecules: By identifying the Label (in this Example, the oligonucleotide label), the pMHCs that bound cells can be identified. Therefore, the oligonucleotides that were comprised within the MHC molecules that were recovered with the cells, were analyzed by quantitative PCR using Label-specific Q-PCR probes. This allowed the identification of pMHCs that bound cells of the cell sample.
   a. Labels derived from sorted cells were analyzed by QPCR as in experiment 1

### Results and conclusions on Example 4

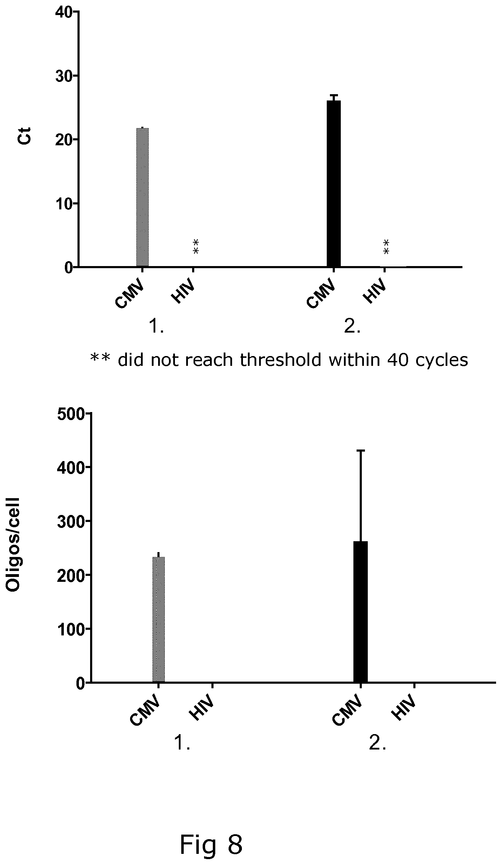

After sorting and qPCR the resultant Ct values confirmed that Labels were successfully recovered and enriched for only when associated with the CMV epitope, while they were not detected when associated with the HIV epitope (figure 8).

It was verified that the 2OS labels were recovered after cellular interaction, sorting and qPCR only if they were associated with positive control reagents.

### Figure 8.

Detection of a CMV specificity amongst negative control barcoded pMHC dextramers. A unique barcode is associated with the positive control reagents in 1., while another unique barcode is associated with the positive control reagents in 2. The spare barcode in each experiment is associated with the HIV negative control reagent. In addition 998x unlabeled negative control reagents are present in both 1. and 2.

**A,** Ct values from multiplex qPCR of the sorted PE-pMHC-dextramer positive cells. Cells were stained with 1. and 2. respectively. Reagents associated with a positive control (CMV) barcode and a negative control (HIV) barcode were present during staining, but the negative control (HIV) barcoded pMHC dextramer was evidently washed out. Approximatly 575 cells were analyzed in each separate qPCR. B. The estimated number of barcodes bound per cell relative to the obtained Ct-values. It is evident that there are some differences in the Ct values shown in B, even though the same number of cells were present in all qPCRs. This is however leveled when the values are normalized in respect to their specific probes. QPCR was run in duplicates, here showing mean ± range of dublicates.

### Example 5

This is an example where the Sample (1) was blood which was modified (1b) to generate Peripheral blood mononuclear cells (PBMCs).

The Backbone (2) was a dextran conjugate with streptavidin and fluorochrome (Dextramer backbone from Immudex).

The MHC molecules (3) are peptide-MHC (pMHC) complexes displaying an 8-10 amino acid peptide-antigen. The MHC molecule was modified (3b) by biotinylation to provide a biotin capture-tag on the MHC molecule. The MHC molecule was purified (2c) by HPLC. The Label (4) was an oligonucleotide. The oligonucleotide label was synthetized (4a) by DNA Technology A/S (Denmark) and was synthetically modified (4b) with a terminal biotin capture-tag. In parts of the example the oligonucleotide label was further modified by annealing to a partially complimentary oligonucleotide label giving rise to a combined oligonucleotide label.

The MHC molecule (5) was synthetized (5a) by attaching MHC molecules in the form of a biotinylated pMHC and labels in the form of a biotin-modified oligonucleotide onto a streptavidin-modified dextran backbone (Dextramer backbone from Immudex, Denmark). The MHC molecule further contains a modification (5b) in the form of a fluorochrome. A library of 110 different MHC molecules were generated wherein individual MHC molecules containing different pMHC were encoded by corresponding individual oligonucleotide labels.

An amount of sample, PBMC's (1b) was incubated with an amount of a library of MHC molecules (5) under conditions (6c) (e.g. incubation time, buffer, pH and temperature) allowing binding of MHC molecules to T cells in the sample.

The cell-bound MHC molecules were separated from the non-cell bound MHC molecules (7) by first a few rounds of washing the PBMC's through centrifugation sedimentation of cells and resuspension in wash buffer followed by Fluorescence Activated Cell Sorting (FACS) of fluorochrome labeled cells. T cells that can efficiently bind MHC molecules will fluoresce because of the fluorochrome comprised within the MHC molecules; T cells that cannot bind MHC molecules will not fluoresce. FACS-sorting leads to enrichment of fluorescent cells, and hence, enrichment of the MHC molecules that bind T cells of the PBMC sample.

FACS isolated cells were subjected to PCR amplification of the oligonucleotide label associated with the MHC molecules bound to cells. Subsequent sequencing of individual DNA fragments generated by the PCR reaction revealed the identity of MHC molecules that bound to the T cells present in the sample.

This experiment thus revealed the peptide-MHC specificity of the T cell receptors of the T cells present in the blood sample.
1. Sample preparation. The cells sample used in this experiment was obtained by mixing blood drawn from 2 different donors BC 260 and 171 (table 6). To provide a titration of the B0702 CMV pp65 TPR responses in a B0702 negative donor sample. 5 fold dilution of BC 260 in 171 was performed i.e. 100, 20, 5, 1, 0.2, 0.04, 0.0125, 0.0025% of BC 260 corresponding to a theoretical frequency of specific cells of 5%, 1%, 0.2%, 0.04%, 0.008%, 0.0016% and 0.00032% B0702 CMV pp65 TPR. Thus, the sensitivity of the method as well as the relevance of the results obtained in the experiment could be evaluated at the end of the experiment, by comparison with data obtained in parallel, using other methods but similar cells.
   a. Acquiring sample: Blood was obtained from the Danish Blood Bank.
   b. Modifying sample:
      i. As in experiment 1
      ii. Mixing of two blood samples as described above
2. Backbone preparation: as in experiment 1
3. MHC molecules preparation: The MHC molecules used in this example were class I MHC-peptide complexes. The individual specifies (allele and epitope combination) were generated as described in experiment 1. Here we used a library of 110 different peptide MHC molecules corresponding to table 10.
   a. Synthesis: As described in experiment 1
      i. Both the MHC heavy chain and B2M was expressed in E.coli as previously described (Hadrup et al. 2009).
      ii. p*MHC monomers were refolded and purified as previously described (Hadrup et al. 2009)
   b. Modification: The p* UV conditional peptide-ligand was exchanged with the peptide antigens to be explored to produce specific peptide MHC monomers.
      i. Peptides (Pepscan Presto) were diluted in phosphate buffered saline (DPBS; Lonza) and mixed to final concentrations of 100µg/ml:200µM (monomer:peptide) in individual wells of 384 well plates. Maximum volumes of 70µl were prepared in the respective well formats. The mixtures were exposed to 366 nm UV light (UV cabinet; CAMAG) for one hour and optionally stored for up to 24 h at 4°C.
   c. Purification: as in experiment 2
4. Label preparation: In this example, 110 different oligonucleotides, of the same length but different sequence, were generated. Each of the oligonuclotides became attached to a specific pMHC, and thus encoded this specific pMHC. The oligonucleotides were biotinylated, allowing easy attachment to the dextran-streptavidin conjugate backbone.
   a. Synthesis: In this example the labels were DNA oligonucleotides which were purchased from DNA Technology (Denmark) and delivered as lyophilized powder. Stock dilutions of 100 µM label were made in nuclease free water and stored at -20 °C. Two types of DNA oligonucleotide labels were used and named 1OS and 2OS respectively.
      i. 120 1OS labels were ordered from DNA Technology as single stranded DNA-oligonucleotides with 5' biotinylation modification. Labels were diluted to working concentrations (640nM) in nuclease free water with 0.1% Tween. See table 9 and 10 for 1OS label sequences.
      ii. A 2OS label system was developed to increase the complexity of a limited number of oligonucleotide sequences by a combinatorial label generation strategy to produce multiple unique labels from a more confined number of label precursors. The strategy, referred to as 2OS, involved annealing and subsequent elongation of two partially complimentary oligonucleotide-sequences that fostered a new unique oligonucleotide-sequences that was applied as a DNA oligonucleotide label (table 9 + 10). E.g. by combining 20 unique oligonucleotide-sequences (A label precursor) that are all partly complementary to 60 other unique oligonucleotide sequences (B label precursor) a combinatorial library of 1,200 different (Ax+By) labels could be produced.
         1. Partly complementary A and B oligonucleotides were annealed to produce a combined A+B oligonucleotide label. An A and a B oligo was mixed as stated in table 3, heated to 65 °C for 2 min and cooled slowly to <35°C in 15-30 min. The annealed A and B oligos were then elongated as stated in table 3.4. Components of the elongation reaction were mixed just before use. After mixing, the reaction was left 5 min at RT to allow elongation of the annealed oligonucleotides. The reagents used for annealing (left) and elongation (right) of partly complementary oligonucleotides is described in Table 3. Reagents marked in italic were from the the Sequenase Version 2.0 DNA Sequencing Kit (Affymetrix #70770).
   b. Modification: All labels were diluted to working concentrations (640nM) in nuclease free water with 0.1% Tween. Elongated oligonucleotide sequence 2OS labels were now treated as 1OS labels.
5. MHC molecules preparation: The MHC molecules (pMHCs) and Labels (oligonucloetides) were attached to the backbone (a dextran-streptavidin-fluorchrome conjugate), to form the MHC molecules, in a way so that a given pMHC is always attached to a given oligonucleotide - maintaining a 1:1 relation between a pMHC and an oligonucleotide.
   a. Synthesis: For preparation of MHC molecules the backbone was labeled in the form of a biotinylated oligonucleotide prior to addition of pMHC.
      i. Creation of MHC molecules were, if not stated otherwise, performed by addition of label in two fold excess over backbone (2:1 label:backbone) and incubated 30 min, 4°C. Binding of label to the backbone (backbone) was always determined after titration when a new batch of backbone and or labels was used. Prior to coupling MHC molecules, pMHC monomers, these were centrifuged 5 min, 3300g. SA conjugate (Dextramer backbone, Immudex) with conjugated streptavidin (SA) and fluorochrome (PE) were aliquoted into new 96 well plates matching the peptide exchange reaction setup. Differences in the procedure for assembling PE. Avoiding the precipitate, MHC molecules were added to the aliquoted SA conjugate and incubated 30 min, RT. Following complex formation, D-biotin (Avidity Bio200) was added together with 0,02% NaN2 in PBS, and incubated 30 min, 4°C. Assembled MHC molecules were stored up to four weeks at 4°C.
   b. Modification: When the total volume of combined panel of MHC molecules exceeded 100 µl per incubation with sample the volume was reduced.
      i. Size exclusion spin columns Vivaspin 500, Sartorius) with a cut-off at 300 kDa were saturated by adding 500µl 2% BSA/PBS and centrifuging 5000g, until the volume had passed through. Subsequently, the columns were washed twice by adding 500µl PBS and centrifuging 5000g until no considerable volume was left in the columns. The combined panel of MHC molecules was added to the spin column and centrifuged 5000g, 4°C until the desired volume resided in the column (approximately 80 µl per incubation with sample).
   c. Purification: The panel of MHC molecules was moved to eppendorph tubes and centrifuged 5 min, 5000g, to sediment any MHC molecules not in solution, before being added to the cells.
6. Incubation of sample and MHC molecules: The cell sample and the MHC molecules were mixed in one container, to allow the MHC molecules to bind the T cells that they recognize.
   a. Amount of sample: 2x10E6 cells in the form of BC's
   b. Amount of MHC molecule
   c. Conditions: All washing of cells refer to centrifugation 5 min, 490 g, with subsequent removal of supernatant. 2x10E6 cells where transferred to individual wells of 96 well plates, washed in barcode-buffer (PBS/0.5% BSA/2 mM EDTA/100 µg/ml herring DNA) and resuspended in this buffer to approximately 20µl per staining. When incubating sample with MHC molecules the cells were incubated with 50nM dasatinib, 30 min, 37°C (Lissina et al. 2009). MHC molecules were centrifuged for 5 min, 3300g, prior to addition to cells. 3 ul of each MHC molecule was required per incubation (1 ug/ml in respect to pMHC). After adding MHC molecules, the cells where incubated 15 min, 37°C. The antibody mixture listed in table 2 were added together with 0.1 µl near- IR-viability dye (Invitrogen L10119) that stains free amines. Antibody staining was essentially as for conventional MHC multimer staining . Cells were incubated 30 min, 4°C before washing of any MHC molecules or antibodies that did not bind to the cells. Cells were subsequently fixed by adding 50 ul 1% paraformaldehyde
7. Enrichment of MHC molecules with desired characteristics: In this Example, the MHC molecules were enriched by using flow cytometry, more specifically, Fluorescence-Activated-Cell-Sorter (FACS). The MHC molecules carry a fluorochrome. Hence, the cells that bind MHC molecules will fluoresce, and can be separated from the cell that do not bind MHC molecules and therefore do not fluoresce, by a FACS sorter. As a result, the MHC molecules that bound to cells will be enriched for.
   a. Apply: Throughout this study two different flow cytometers where used for acquisition. A BD FACSCanto II equipped with three lasers (488 nm blue, 633nm red and 405 violet) and a BD LSR II cytometer equipped with five lasers. Only four lasers on the LSR II were used throughout this study (488 nm blue laser, 640 nm red laser, 355 nm UV laser and 405 nm violet laser). Additionally cells were sorted on BD FACSAria and FACSAria II, equipped with three lasers (488 nm blue, 633nm red and 405 violet). All flow cytometry data analyses used the BD FACSDiva software version 6.1.2.
      i. The following gating strategy was used. All initial gatings of CD8 positive cells were performed alike. Lymphocytes were identified in a FSC/SSC plot. Additional gating on single cells (FSC-A/FSC-H), live cells (near-IR-viability dye negative), and dump-channel negative/CD8 positive cells (FITC/PerCP) where used to define the CD8 T cell population.
   b. Wash: Cells were washed twice in barcode-buffer where after the cells were ready for flow cytometric acquisition. Optionally cells were fixed in 1% paraformaldehyde O.N., 4°C, and washed twice in FACS buffer or barcode-buffer. Fixed cells were stored for up to a week at 4°C.
   c. Separate: Optionally cells were acquired up to one week after fixation in 1% paraformaldehyde. The multimer positive cells were sorted into tubes that had been pre-saturated for 2h-O.N. in 2% BSA and contained 200 µl barcode-buffer to increase the stability of the oligonucleotides that followed with the sorted cells. The sorted multimer positive cells were centrifuged 5 min, 5000 g, to allow removal of all excess buffer. Cells were stored at -80 °C.
      i. Gates were drawn to define the positive events from the single conjugated fluorochrome, i.e. PE or APC.
      ii. The capacity of pMHC dextramers were evaluated based on the mean fluorescent intensity (MFI) or the stain index (SI). SI is a measure of population separation, taken into account also potential effects on the negative population (background) and the spread of the background:
8. Identification of enriched MHC molecule: By identifying the Label (in this Example, the oligonucleotide label), the pMHCs that bound cells can be identified.
   Therefore, the oligonucleotides that were comprised within the MHC molecule that were recovered with the cells, were sequenced. This allowed the identification of pMHCs that bound cells of the cell sample.
   a. Labels derived from sorted cells were amplified by PCR prior to sequencing. See table 4 for composition of the PCR. PCR was performed with the kit: Taq PCR Master Mix Kit (Qiagen, #201443). The thermal profile is listed in table 5. PCR was run on the thermal cycler: GeneAmp, PCR System 9700 (Applied Biosystem). PCR products were visualized, after gel electrophoresis on a Bio-Rad Gel Doc EZ Imager.
      i. The forward and reverse primer included adaptors for the sequencing reaction (A-key and P1-key respectively which are compatible with Ion Torren sequencing, Life Technologies).
      ii. Moreover the forward primer carried a sample-identification barcode (table 8). Labels on sorted cells and their associated MHC molecules derived from individual samples were amplified with primers holding a specific sample-identification sequence (Table 8). This facilitated distribution of sequence reads derived from every single sample. Additionally, the input of concentrated panels of MHC molecules (before mixing with cells) were allocated a sample-identification barcode through PCR (referred to as the panel-input). Sequencing of the panel-input would allow normalization of the analyzed sequence output.
      iii. Positive sequence reads were aligned to sequences that read from the sample-barcode-identity at the 5'-end all the way through the pMHC-barcode-identity. The numbers of reads were normalized according to the total number of reads that mapped to the same sample-barcode-identity and according to the panel-input reads.
      Deconvolute label on MHC molecule
   b. Sequencing of DNA oligonucleotide labels was carried out on a 314 Ion Torrent chip (GeneDx). Adaptors were introduced via primers during PCR (refer to table 8 for adaptor sequences)
      i. A sequence database was created consisting of the possible combinations of 15 sample-identification barcodes and 358 pMHC barcodes (118 1OS + 240 2OS), together with the primer and annealing sequences from both the 1OS and 2OS systems. This accumulated to 5370 sequences that could be expected from a sequencing run. Each sequencing read was then used to search the database for alignments, using the nucleotide BLAST algorithm, with a match reward of 1, mismatch reward of -2 and a gap cost of 2 for both opening and extending a gap. In this way sequencing errors were penalized equally, whether a base was miscalled or inserted/deleted in the sequencing read compared to the actual sequence.
      ii. Alignments were discarded by the following criteria:
         1. E-value > 1e-12; insufficient length of alignment (should be greater than 60 or 102 bases for the 1OS and 2OS systems, respectively)
         2. Start position in subject sequence larger than 2, i.e. fewer than 5 out of 6 bases in the unique part of the sample-identification barcode was included in the alignment.
         3. If multiple alignments could still be found for any sequencing read, only the alignment with the best percent identity was kept. Finally, the number of reads mapping to each barcode in the database was counted.
      iii. Identifying overrepresented barcodes: Relative read counts were calculated by normalizing each read to the total read count mapping to the same sample-identity barcode. The relative read counts were then used to calculate the fold change per barcode compared to the control sample-barcode input (barcoded detection-molecule panel that was not mixed with cells). Significantly overrepresented barcodes were identified using a 2-sample test for equality of proportions on the raw read counts in a sample versus the control-barcode input, and p-values were corrected for multiple testing using the Benjamini-Hochberg FDR method.

### Result of example 5:

This example shows the feasibility for detection of antigen responsive T-cell in a large mixture of different pMHC multimer (MHC molecules). We show the sensitivity of the barcode-labelled MHC multimers being at least able to detect 0.00032% of specific T-cell out of CD8 T cells. We find exact correlation withprevious described (low throughput) methods.

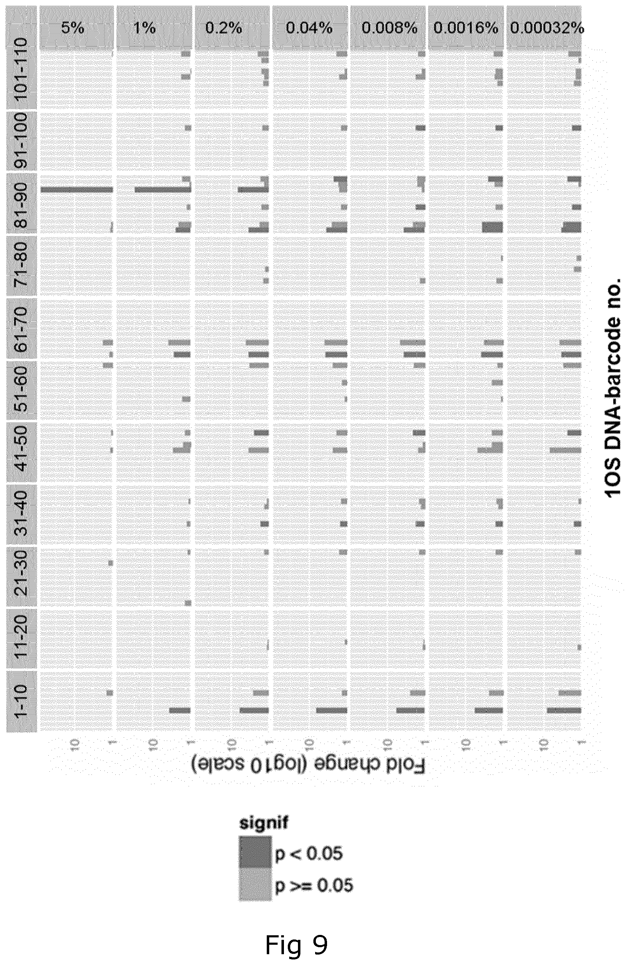

Figure 9. Schematic presentations of the number of specific 1OS barcode reads mapped to seven different samples. A 5% B7 CMV pp65 TPR response (barcode 88) were spiked into a HLA-B7 negative BC in fivefold dilutions, creating seven samples (5%, 1%, 0.2%, 0.04%, 0.008%, 0.0016% and 0.00032%). This BC has a population of A11 EBV-EBNA4 specific T cell (corresponding to barcode 4). Samples were stained with the same panel comprising 110 differently 1OS barcoded-pMHC-dextramers. The bars show the total reads normalized to the input panel in each sample. Experiments were performed in duplicate. Here showing mean.

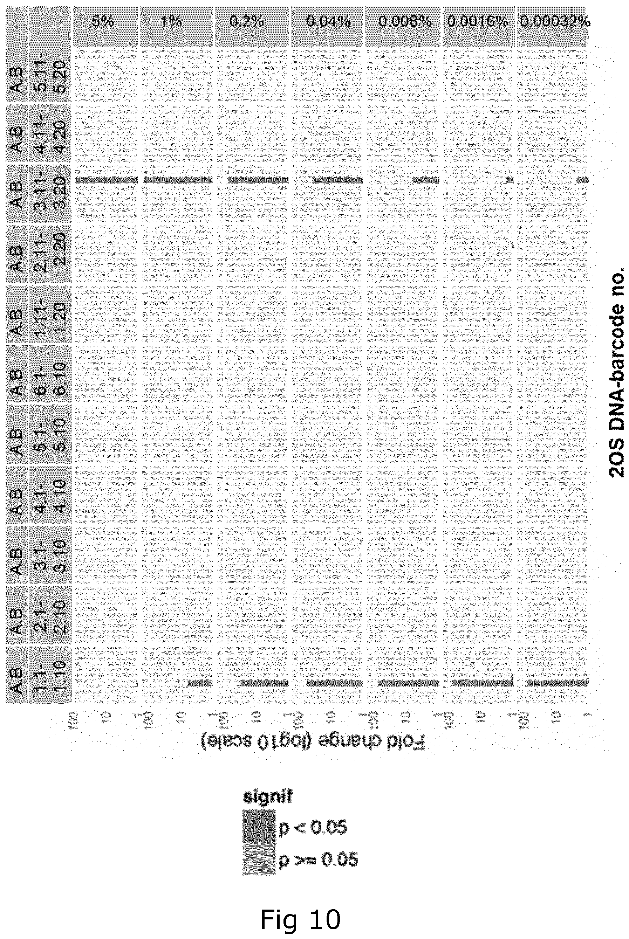

Figure 10. Schematic presentations of the number of specific 2OS barcode reads mapped to seven different samples. A 5% B7 CMV pp65 TPR response (barcode A3B18) were spiked into a HLA-B7 negative BC in fivefold dilutions, creating seven samples (5%, 1%, 0.2%, 0.04%, 0.008%, 0.0016% and 0.00032%). This BC has a population of A11 EBV-EBNA4 specific T cell (corresponding to barcode A1B4). Samples were stained with the same panel comprising 110 differently 2OS barcoded-pMHC-dextramers. The bars show the total reads normalized to the input panel in each sample. Experiments were performed in duplicate. Here showing mean.

### Example 6:

Examples 6 is conducted exactly as examples 5, with the only difference that we have used a different sample. Here we detect antigen responsive T-cells in 5 different donor blood samples.

### Results example 6:

This example shows the feasibility to detect numerous different specificities in different donor samples using DNA barcode labelled MHC multimers. Obtained data show the feasibility for high-throughput screening of T-cell reactivity in numerous donor to assess immune reactivity associated with disease development, vaccination, infection etc.

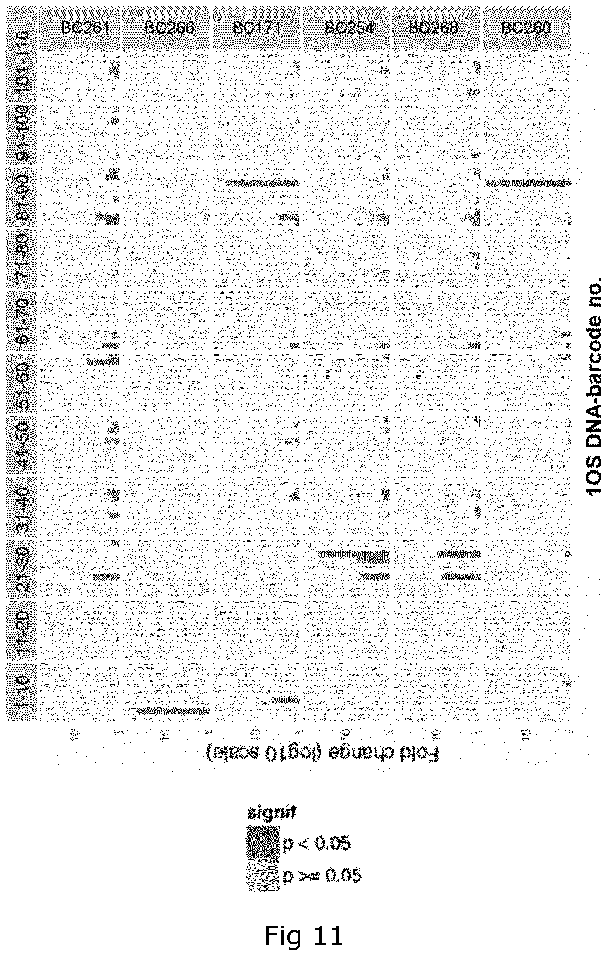

Figure 11. A schematic presentations of the number of specific 1OS barcode reads mapped to six different samples. Six BCs were stained with the same panel comprising 110 differently 1OS barcoded-pMHC-dextramers. Bar charts show the total reads normalized to the input panel in each sample (p<0.05). Each pie chart show significant (p<0.01) reads mapped to that sample.

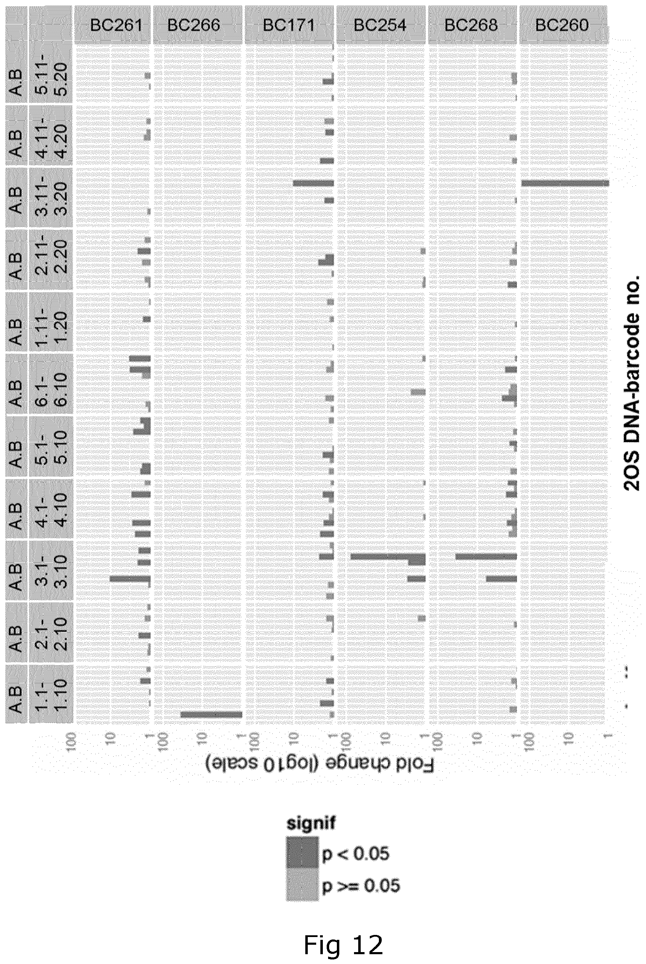

Figure 12. Schematic presentations of the number of specific 2OS barcode reads mapped to six different samples. Six BCs were stained with the same panel comprising 110 differently 2OS barcoded-pMHC-dextramers. Bar charts show the total reads normalized to the input panel in each sample (p<0.05).

### Tables:

**Table 1: A listing of reagents required for production of pMHC multimers produced from 100 µg/ml exchange reaction. The amounts of the respective reagents used for staining 1x106-2x106 cells in 100 µl are also specified.**

| | **SA conjugate/µl exchange** | **D-biotin** | **End: pMHC TET** | **Amount per staining** |
|---|---|---|---|---|
| **SA** | 0.092 µl (0.1 µg/ml) | 28 µM | 100 µg/ml | 1 µl |
| **PE** | 1.32 µl | 12.6 µM | 44 µg/ml | 3 µl |
| **APC** | 0.73 µl | 9.78 µM | 24.25 µg/ml | 3 µl |

**Table 2: A listing of the components in the antibody mixture added after incubation with MHC molecules or after staining with conventional MHC multimers.**

| **Target** | **Conjugate** | **Amount (µl)** | **Source** |
|---|---|---|---|
| CD8 | PerCP | **2** | Invitrogen MHCD0831 |
| CD4 | FITC | 1.25 | BD bioscience 345768 |
| CD14 | FITC | 3.13 | BD bioscience 345784 |
| CD16 | FITC | 6.25 | BD bioscience 335035 |
| CD19 | FITC | 2.50 | BD bioscience 345776 |
| CD40 | FITC | 1.56 | Serotec MCA1590F |

**Table 3: The reagents used for annealing (left) and elongation (right) of partly complementary oligonucleotides. Reagents marked in italic were from the the Sequenase Version 2.0 DNA Sequencing Kit (Affymetrix #70770).**

| **Annealing reaction** | **(10 µl)** | **Elongation reaction** | **(15.5 µl)** |
|---|---|---|---|
| Oligo A (100 µM) | 2.6 µl | Annealing reaction | 10 µl |
| Oligo B (100 µM) | 5.4 µl | 0.1M DTT | 1 µl |
| *Sequenase reaction buffer* | 2 µl | H₂O | 0.5 µl |
| | | 8x diluted *Sequenase polymerase* | 2 µl |
| | | 5x diluted *Sequence extension mixture* | 2 µl |

**Table 4: The PCR Master mix applied prior to sequencing of labels on MHC molecules associated with sorted cells. The forward and reverse primer included adaptors for the sequencing reaction (A-key and P1-key respectively). Moreover the forward primer carried a sample-identification sequence (table 8).**

| **Component** | **Volume per sample (µl)** |
|---|---|
| Master Mix | 25 |
| Forward Primer (5 µM) | 3 |
| Reverse Primer (5 µM) | 3 |
| Nuclease free H₂O | 9 |
| Template | 10 |

**Table 5: The thermal profile applied for amplification of labels on MHC molecules associated with sorted cells. 36 cycles were applied if >1,000 cells were sorted while 38 cycles were applied if <1,000 cells were sorted.**

| **Temperature (°C)** | | **Time** | **No. of cycles** |
|---|---|---|---|
| **-** | 95 | 10 min | 1 |
| - | 95 | 30 s | 36-38 |
| - | 60 | 45 s | |
| - | 72 | 30 s | |
| - | 72 | 4 min | 1 |
| - | 4 | ∞ | |

**Table 6. BCs included in the experiments 3-6. The virus specificities detected by combinatorial encoding of conventional MHC multimers with 25 virus peptides. The frequency of each response is listed along with the 1OS and 2OS label numbers appointed in the experiment.**

| | **Epitope** | **Freq. (%)** | **1OS** | **2OS** |
|---|---|---|---|---|
| **BC261** | A2 FLU MP 58-66 GIL | 0.1249 | 24 | A3B4 |
| | A3 EBV EBNA 3a RLR | 0.0258 | 60 | A6B10 |
| | A2 EBV LMP2 FLY | 0.0075 | 27 | A3B7 |
| **BC266** | A1 CMV pp65 YSE | 0.0859 | 1 | A1B1 |
| | A1 FLU BP-VSD | 0.0628 | 3 | A1B3 |
| **BC171** | A11 EBV-EBNA4 | 0.3 | 4 | A1B4 |
| | A3 CMV pp150 TVY | 0.015 | 61 | A1B11 |
| **BC254** | A2 FLU MP 58-66 GIL | 0.0522 | 24 | A3B4 |
| | A2 EBV LMP2 FLY | 0.014 | 27 | A3B7 |
| | A2 CMV pp65 NLV | 1.1279 | 28 | A3B8 |
| **BC268** | A2 FLU MP 58-66 GIL | 0.2523 | 24 | A3B4 |
| | A2 CMV pp65 NLV | 05445 | 28 | A3B8 |
| **BC260** | A2 FLU MP 58-66 GIL | 0.0456 | 24 | A3B4 |
| | A2 CMV pp65 NLV | 0.134 | 28 | A3B8 |
| | B7 CMV pp65 TPR | 4.5395 | 88 | A3B18 |

**Table 7: Test Oligos with different end modifications**

| | | |
|---|---|---|
| 'b' = Biotin-TEG 5' modification | | |
| 'h' = HEG (terminal modifications) | | |
| Forward-01 | | GAGATACGTTGACCTCGTTG |
| Reverse-01 | | ATGCAACCAAGAGCTTAAGT |
| Reverse-03 | | hATGCAACCAAGAGCTTAAGT |
| TestOligo-01 | | |
| bGAGATACGTTGACCTCGTTGAANNNNNNTCTATCCATTCCATCCAGCTCACTTAAGC TCTTGGTTGCAT | | |
| TestOligo-02 | | |
| bhGAGATACGTTGACCTCGTTGAANNNNNNTCTATCCATTCCATCCAGCTCACTTAAG CTCTTGGTTGCAT | | |
| TestOligo-03 | | |
| bhGAGATACGTTGACCTCGTTGAANNNNNNTCTATCCATTCCATCCAGCTCACTTAAG CTCTTGGTTGCATh | | |
| TestOligo-04 | | |
| bhGAGATACGTTGACCTCGTTGAANNNNNNTCTTGAACTATGAATCGTCTCACTTAAG CTCTTGGTTGCATh | | |

| | | |
|---|---|---|
| TestOligo-05 | | |
| bhGAGATACGTTGACCTCGTTGAANNNNNNTCTATAGGTGTCTACTACCTCACTTAAG CTCTTGGTTGCATh | | |
| TestOligo-06 | | |
| bhGAGATACGTTGACCTCGTTGAANNNNNNTCTTTATTGGAGAGCACGCTCACTTAAG CTCTTGGTTGCATh | | |
| Probe-03 Tm 64,9 | 8TCTATCCATTCCATCCAGCTC7 | 8 = FAM; 7 = BHQ-1-plus |
| Probe-04 Tm 57,3 | 8TCTTGAACTATGAATCGTCTC7 | 8 = FAM; 7 = BHQ-1-plus |
| Probe-05 Tm 58,5 | 9TCTATAGGTGTCTACTACCTC7 | 9 = HEX; 7 = BHQ-1-plus |
| Probe-06 Tm 60,9 | 2TCTTTATTGGAGAGCACGCTC1 | 2 = Cy5; 1 = BHQ-2-plus |
| LNA-3 TCTATCCATTCCATCCAGC | | 8 = FAM; 7 = BHQ-1-plus |
| LNA-4 TCT[+T][+G][+A]AC[+T][+A]TG[+A][+A][+T]CGTC | | 8 = FAM; 7 = BHQ-1-plus |
| LNA-5 TCT[+A][+T][+A]GG[+T][+G]TC[+T][+A][+C]TACC | | 9 = HEX; 7 = BHQ-1-plus |
| LNA-6 TCT[+T][+T][+A]TT[+G][+G]AG[+A][+G][+C]ACGC | | 2 = Cy5; 1 = BHQ-2-plus |

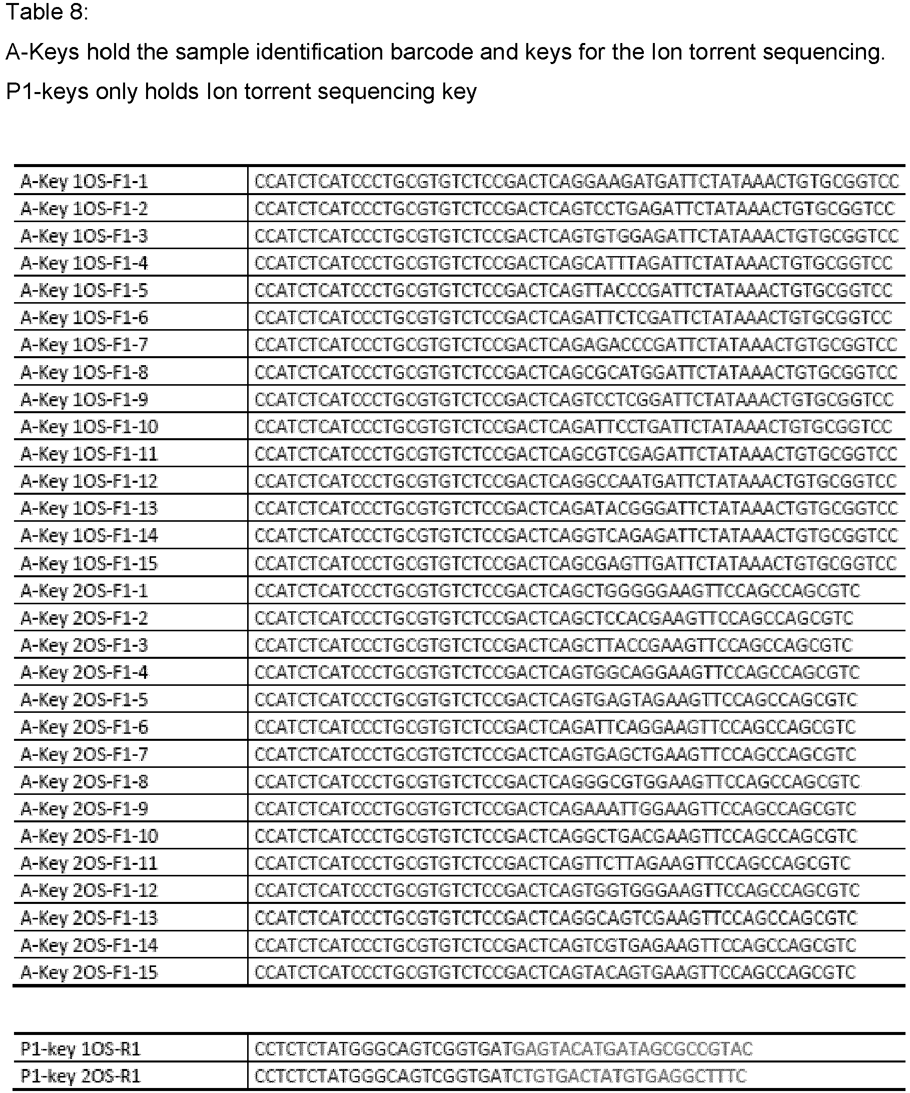

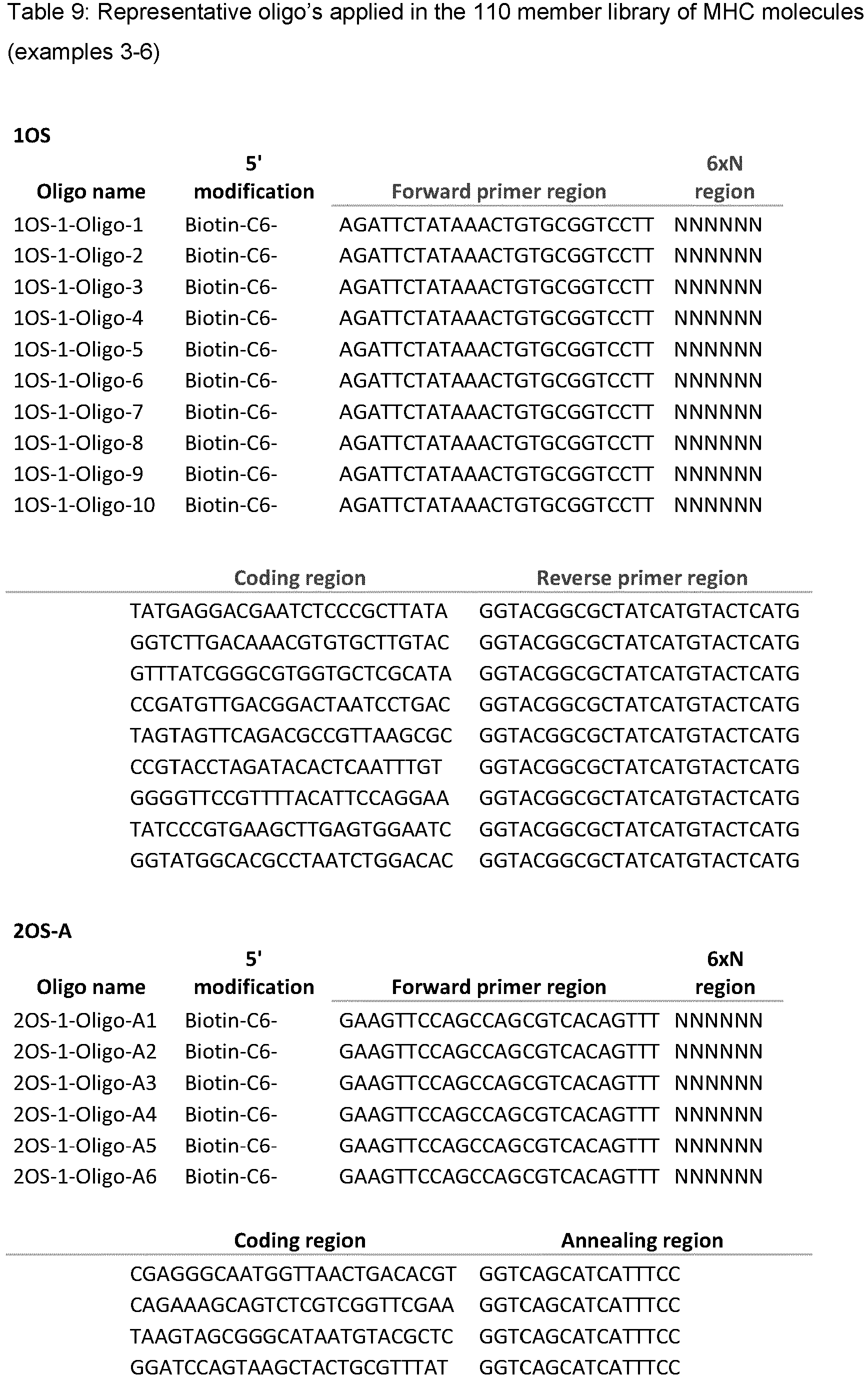

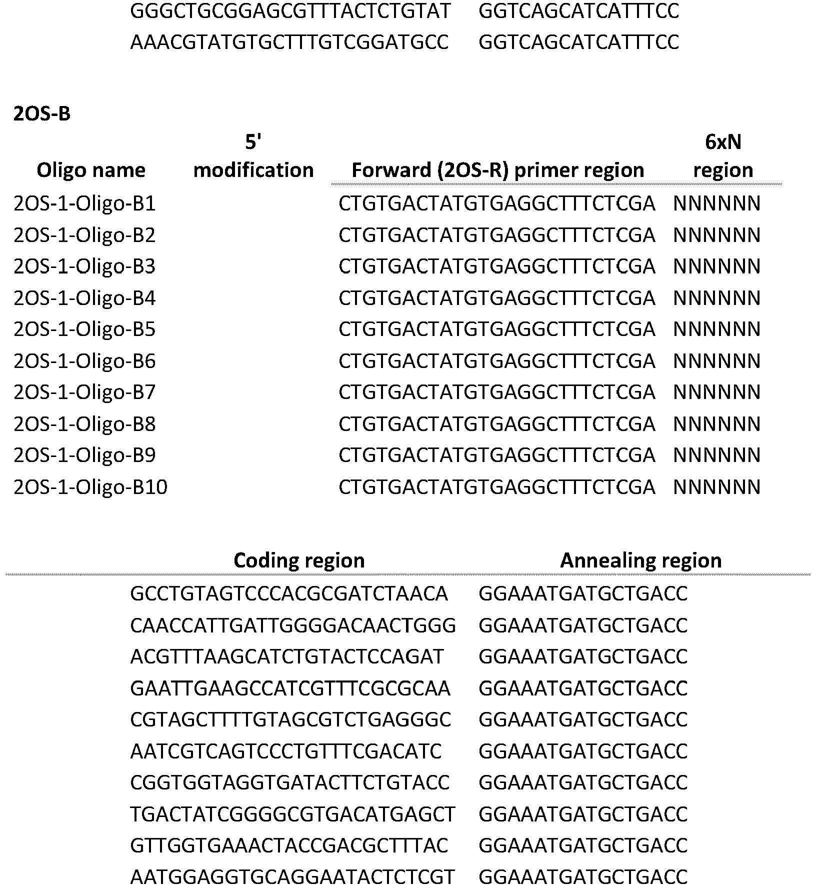

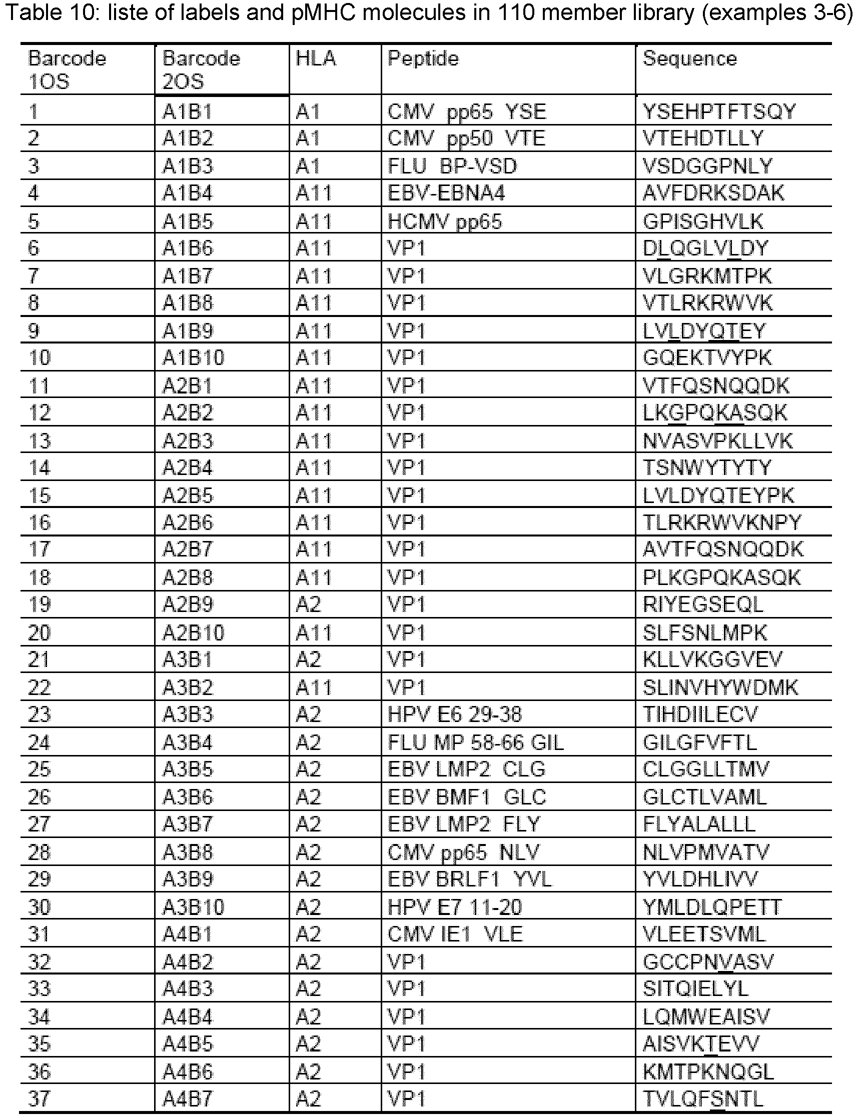

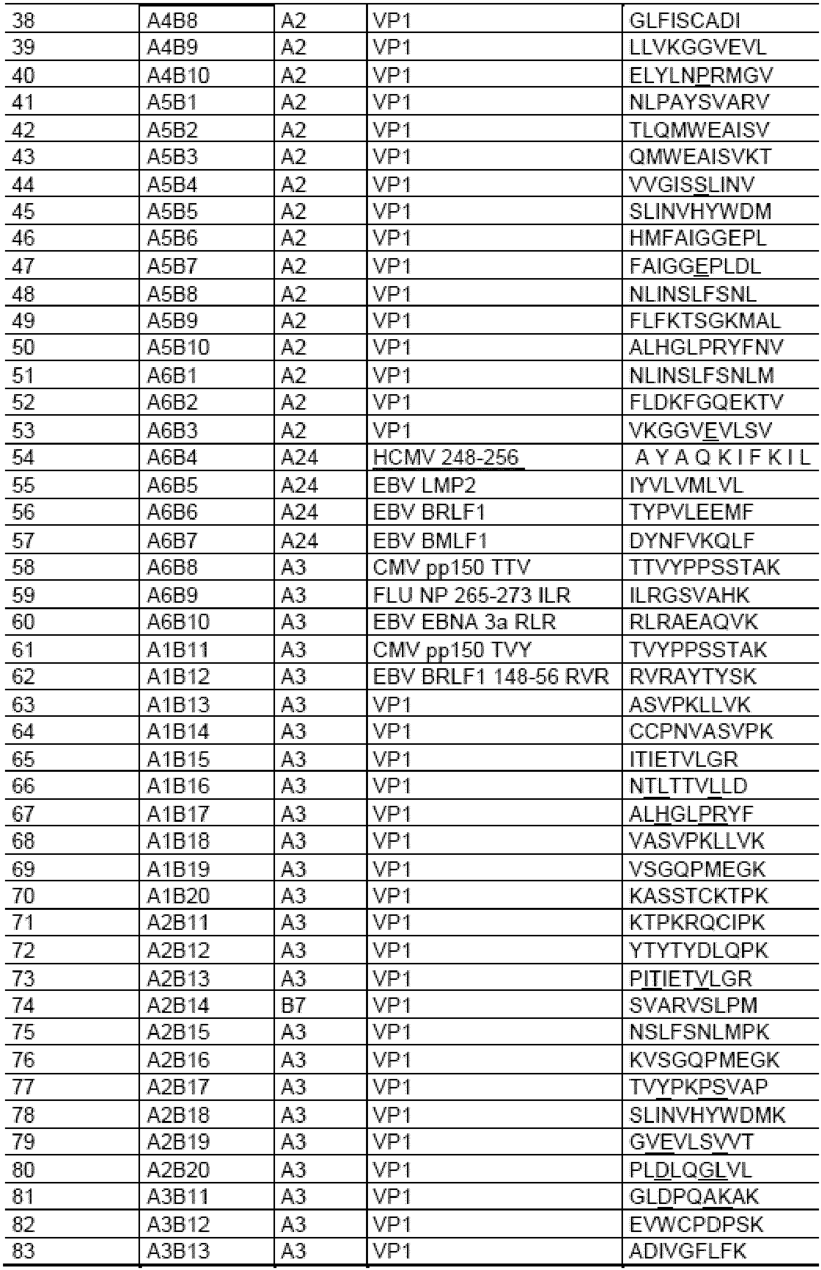

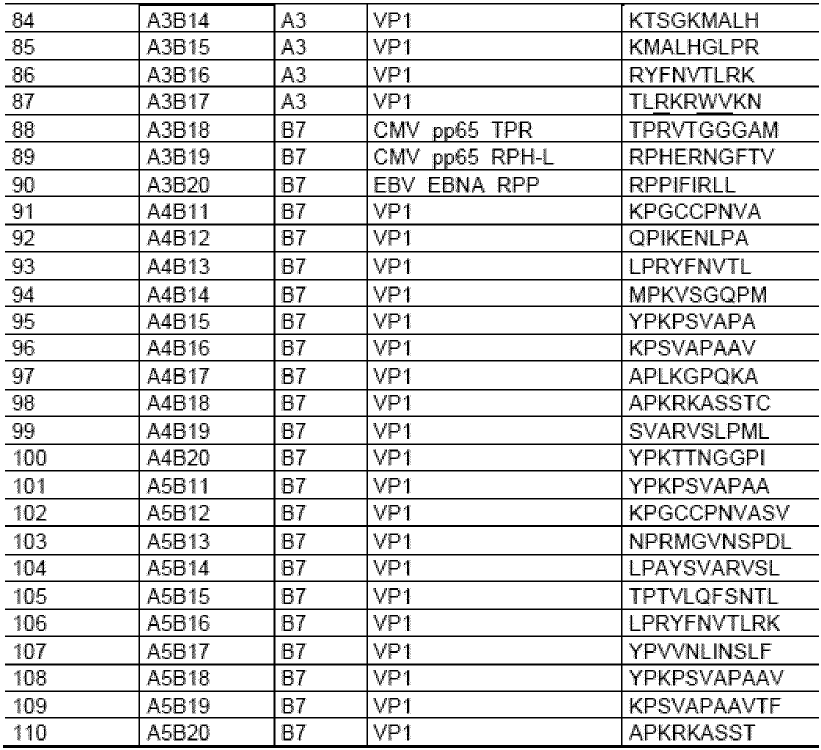

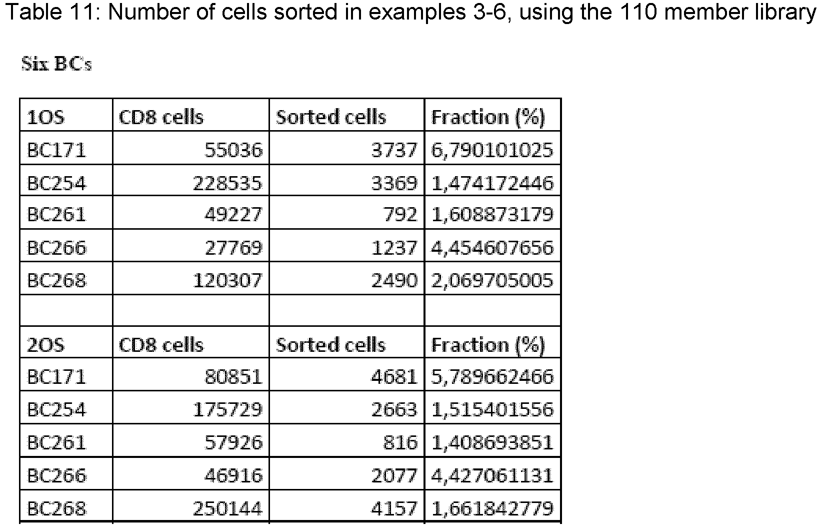

**Table 12:**

| |
|---|
| Abbreviations |
| 1OS Single oligo system |
| 2OS Two oligo system |
| AIRE Autoimmune regulator |
| APC Allophycocyanin |
| Barcode oligonucleotide sequence |
| BC Buffy coat |
| B cell B lymphocyte |
| BSA Bovine Serum albumin |
| CD Cluster of differentiation |
| CDR Complementary-determining regions |
| CMV Cytomegalovirus |
| Ct Cross threshold |
| CTL Cytotoxic T lymphocyte |
| CyTOF Cytometry by time-of-flight |
| DC Dendritic cells |
| DMSO Dimethyl sulfoxide |
| dT Thymidine backbone |
| EBV Epstein-Barr virus |
| EDTA Ethylenediaminetetraacetic acid |
| ELISPOT enzyme-linked immunospot |
| ER Endoplasmatic reticulum |
| FACS Fluorescence activated cell sorting |
| FBS Fetal Bovine Serum |
| FCS Fetal calf serum |
| FITC Fluorescein isothiocyanate |
| HEG Hexaethylene glycol |
| HIV Human immunodeficiency virus |
| HLA Human leukocyte antigen |
| HPLC High-performance liquid chromatography |
| IFN Interferon |
| li Invariant chain |
| IL Interleukin |
| MHC Major Histocompatibility Complex |
| N6 Randon six nucleotides |
| NIR Near-infrared |
| nt Nucleotide |
| O.N. Over night |
| PBMC Peripheral blood mononuclear cell |
| PBS Phosphate buffered saline |
| PCR Polymerase chain reaction v |
| PE R-phycoerythrin |
| PerCP Peridinin chlorophyll |
| p* UV-conditional peptide |
| PBS Phosphate buffered saline |
| pMHC Peptide-Major histocompatibility complex |
| PCR Polymerase chain reaction |
| qPCR Quantitative polymerase chain reaction |
| RAG1/RAG2 Recombinant activating genes |
| RT Room temperature |
| SA Streptavidin |
| SI Stain index |
| TAP1/TAP2 Transporter associated with antigen processing |
| T cell T lymphocyte |
| TCR T cell receptor |
| TEG Triethylene glycol |
| TET Tetramers |
| Th T helper cells |
| TIL Tumor Infiltrating Lymphocyte |
| Tm Melting temperature |
| TNF Tumor necrosis factor |
| Treg T regulatory cells vi vii |

### References

1. Altman JD, Moss PA, Goulder PJ, Barouch DH, McHeyzer-Williams MG, Bell JI, et al. Phenotypic analysis of antigen-specific T lymphocytes. Science. 1996;274:94-6.
2. Davis MM, Bjorkman PJ. T-cell antigen receptor genes and T-cell recognition. Nature. 1988;334:395-402.
3. Robins HS, Campregher P V, Srivastava SK, Wacher A, Turtle CJ, Kahsai O, et al. Comprehensive assessment of T-cell receptor beta-chain diversity in alphabeta T cells. Blood. 2009;114:4099-107.
4. Hadrup SR, Bakker AH, Shu CJ, Andersen RS, van VJ, Hombrink P, et al. Parallel detection of antigen-specific T-cell responses by multidimensional encoding of MHC multimers. Nature Methods. 2009;6:520-6.
5. Andersen RS, Kvistborg P, Morch TF, Pedersen NW, Lyngaa R, Bakker AH, et al. Parallel detection of antigen-specific T-cell responses by combinatorial encoding of MHC multimers. NatProtoc. 2012
6. Newell EW, Sigal N, Nair N, Kidd B a, Greenberg HB, Davis MM. Combinatorial tetramer staining and mass cytometry analysis facilitate T-cell epitope mapping and characterization. Nat Biotechnol. 2013;1-9.
7. Soen Y, Chen DS, Kraft DL, Davis MM, Brown PO. Detection and characterization of cellular immune responses using peptide-MHC microarrays. PLoSBiol. 2003; 1:429-38.
8. Stone JD, Demkowicz Jr. WE, Stern LJ. HLA-restricted epitope identification and detection of functional T cell responses by using MHC-peptide and costimulatory microarrays. ProcNatlAcadSciUSA. 2005;102:3744-9.
9. Newell EW, Davis MM. Beyond model antigens: high-dimensional methods for the analysis of antigen-specific T cells. Nat Biotechnol. 2014;32.
10. Dössinger G, Bunse M, Bet J, Albrecht J, Paszkiewicz PJ, Weißbrich B, et al. MHC multimer-guided and cell culture-independent isolation of functional T cell receptors from single cells facilitates TCR identification for immunotherapy. PLoS One. 2013;8:e61384.
11. Cha E, Klinger M, Hou Y, Cummings C, Ribas A, Faham M, et al. Improved Survival with T Cell Clonotype Stability After Anti-CTLA-4 Treatment in Cancer Patients. Sci Transl Med. 2014;6:238ra70.
12. Robert L, Tsoi J, Wang X, Emerson RO, Homet B, Chodon T, et al. CTLA4 blockade broadens the peripheral T cell receptor repertoire. Clin Cancer Res. 2014
13. Morgan RA, Dudley ME, Wunderlich JR, Hughes MS, Yang JC, Sherry RM, et al. Cancer Regression in Patients After Transfer of Genetically Engineered Lymphocytes. Science. 2006.
14. Pannetier C, Even J, Kourilsky P. T-cell repertoire diversity and clonal expansions in normal and clinical samples. ImmunolToday. 1995;16:176-81.
15. Cameron BJ, Gerry AB, Dukes J, Harper J V, Kannan V, Bianchi FC, et al. Identification of a Titin-derived HLA-A1-presented peptide as a cross-reactive target for engineered MAGE A3-directed T cells. Sci Transl Med. 2013;5:197ra103.
16. Linette GP, Stadtmauer E a, Maus M V, Rapoport AP, Levine BL, Emery L, et al. Cardiovascular toxicity and titin cross-reactivity of affinity-enhanced T cells in myeloma and melanoma. Blood. 2013;122:863-71.

## Claims

1. A method for detecting antigen responsive T cells in a sample comprising:
i) providing a composition comprising different subsets of multimeric MHCs,
wherein each multimeric MHC comprises i) two or more given MHC-peptide molecules linked to a backbone molecule, and ii) at least one nucleic acid molecule linked to said backbone molecule, wherein said at least one nucleic acid molecule comprises a 5' first primer region, a central stretch of nucleic acids (barcode region) and a 3' second primer region, wherein said barcode region serves as a specific label for said two or more given MHC-peptide molecules;
wherein each subset of multimeric MHC has a unique barcode region specific for the given MHC-peptide molecule; and
ii) contacting said multimeric MHCs with said sample; and
iii) removing unbound multimeric MHCs before amplification by washing and spinning; and
iv) detecting binding of the multimeric MHCs to said antigen responsive T cells, thereby detecting T cells responsive to an antigen present in said multimeric MHCs,
wherein said binding of said multimeric MHCs to said antigen responsive T cells is detected by
a) amplifying by PCR the barcode region of said nucleic acid molecules linked to the cell-bound multimeric MHCs , and
b) sequencing of said amplified barcode regions.

2. The method according to claim 1, wherein the primer regions of said nucleic acid molecules are identical for each subset of multimeric MHC, and wherein in step iv) a) the barcode regions of said nucleic acid molecules are amplified using one primer set.

3. The method according to any of claims 1 to 2, wherein said method further comprises sorting of one or more populations of antigen responsive T cells before amplification.

4. The method according to any of claims 1 to 3, wherein said method further comprises single-cell sorting before amplification.

5. The method according to any of claims 3 to 4, wherein said cell sorting comprises a method selected from the group consisting of flow cytometry such as FACS; magnetic-bead based selection; size-exclusion; gradient centrifugation; column attachment and gel-filtration.

6. The method according to any of the preceding claims, wherein said sequencing is selected from deep sequencing, high-throughput sequencing and next generation sequencing.

7. The method according to any of the preceding claims, wherein said detection of binding of antigen responsive cells includes comparing measured values to a reference level, such as a negative control and/or total level of response.

8. The method according to any of the preceding claims, wherein
a. the specific T-cell frequency is calculated by comparing the frequency of barcode-sequence reads to the number of sorted T-cells, or
b. the relative contribution of multiple different peptide-MHC complexes bound to a single T cell is determined by the number of barcode-reads of the given sequences.

9. The method according to any of claims 4 to 8, wherein the antigen specificity of a given sorted single cell is determined in combination with single cell TCR sequencing.

10. The method according to any of the preceding claims, wherein the affinity and binding motif of a given TCR is determined.

11. The method according to any of the preceding claims, wherein the at least one nucleic acid molecule
a. has a length of 20-200 nucleotides, such as 20-150, such as 20-100, such as 30-100, such as 30-80, such as 30-50 nucleotides, and/or
b. comprises or consists of DNA, RNA, and/or artificial nucleotides such as PNA or LNA, such as comprises or consists of DNA or RNA; and and/or
c. comprises a central stretch of nucleic acids (barcode region) ranging from 10 to more than 50 nucleotides.

12. The method according to any of the preceding claims, wherein the backbone further comprises one or more selectable labels, such as one or more labels selected from the group consisting of fluorescent labels, His-tags and metal-ion tags.

13. The method according to any of the preceding claims, wherein the backbone molecule is selected from the group consisting of polysaccharides, including glucans such as dextran, a streptavidin and a streptamer multimer.

14. The method according to any of the preceding claims, wherein said multimeric MHC is composed of at least four given MHC-peptide molecules, such as at least eight, such as at least ten, such as 2-30, such as 2-20, such as 2-10, such as 4-10 or such as 15-30 given MHC-peptide molecules.

15. The method according to any of the preceding claims, wherein the MHC is selected from the group consisting of a class I MHC, a class II MHC, a CD1, and other MHC-like molecules.

16. The method according to any of the preceding claims, wherein said method is for detecting multiple different antigen responsive cells in a sample, such as in a single sample.

17. The method according to any of the preceding claims, wherein the sample is selected from the group consisting of a blood sample, such as a peripheral blood sample; a blood derived sample; a tissue sample, and a body fluid, such as spinal fluid or saliva.

## Patentansprüche

1. Verfahren zum Nachweisen antigenreaktiver T-Zellen in einer Probe, umfassend:
i) Bereitstellen einer Zusammensetzung, die verschiedene Teilmengen von multimeren MHC umfasst, wobei jeder multimere MHC i) zwei oder mehr bestimmte MHC-Peptidmoleküle, die an ein Grundmolekül gebunden sind, und ii) mindestens ein Nukleinsäuremolekül, das an das Grundmolekül gebunden ist, umfasst, wobei das mindestens eine Nukleinsäuremolekül eine erste 5'-Primerregion, eine zentrale Nukleinsäurestrecke (Barcoderegion) und eine zweite 3'-Primerregion umfasst, wobei die Barcoderegion als spezifische Markierung für die zwei oder mehr bestimmten MHC-Peptidmoleküle dient;
wobei jede Teilmenge des multimeren MHC eine eindeutige Barcoderegion aufweist, die für das bestimmte MHC-Peptidmolekül spezifisch ist; und
ii) Inkontaktbringen der multimeren MHC mit der Probe; und
iii) Entfernen ungebundener multimerer MHC vor der Amplifikation durch Waschen und Schleudern; und
iv) Nachweisen der Bindung der multimeren MHC an die antigenreaktiven T-Zellen, wodurch auf ein in den multimeren MHC vorhandenes Antigen reagierende T-Zellen nachgewiesen werden, wobei die Bindung der multimeren MHC an die antigenreaktiven T-Zellen nachgewiesen wird durch
a) Amplifizieren der an die zellgebundenen multimeren MHC gebundenen Barcoderegion der Nukleinsäuremoleküle durch PCR und
b) Sequenzieren der amplifizierten Barcoderegionen.

2. Verfahren nach Anspruch 1, wobei die Primerregionen der Nukleinsäuremoleküle für jede Teilmenge eines multimeren MHC identisch sind und wobei in Schritt iv) a) die Barcoderegionen der Nukleinsäuremoleküle unter Verwendung eines Primersatzes amplifiziert werden.

3. Verfahren nach einem der Ansprüche 1 bis 2, wobei das Verfahren ferner Sortieren einer oder mehrerer Populationen von antigenreaktiven T-Zellen vor der Amplifikation umfasst.

4. Verfahren nach einem der Ansprüche 1 bis 3, wobei das Verfahren ferner Einzelzellensortieren vor der Amplifikation umfasst.

5. Verfahren nach einem der Ansprüche 3 bis 4, wobei das Zellsortieren ein Verfahren umfasst, das aus der Gruppe ausgewählt ist, die aus Durchflusszytometrie wie etwa FACS; Magnetkügelchen-basierter Selektion; Größenausschluss; Gradientenzentrifugation; Säulenanlagerung und Gelfiltration besteht.

6. Verfahren nach einem der vorhergehenden Ansprüche, wobei das Sequenzieren aus Tiefensequenzierung, Hochdurchsatz-Sequenzierung und Next-Generation-Sequenzierung ausgewählt ist.

7. Verfahren nach einem der vorhergehenden Ansprüche, wobei der Nachweis der Bindung von antigenreaktiven Zellen Vergleichen von Messwerten mit einem Referenzpegel, wie einer Negativkontrolle und/oder einem Gesamtansprechpegel, beinhaltet.

8. Verfahren nach einem der vorhergehenden Ansprüche, wobei
a. die spezifische T-Zellenhäufigkeit durch Vergleichen der Häufigkeit von Barcodesequenz-Lesevorgängen mit der Anzahl sortierter T-Zellen berechnet wird, oder
b. der relative Beitrag von mehreren verschiedenen Peptid-MHC-Komplexen, die an eine einzige T-Zelle gebunden sind, durch die Anzahl der Barcode-Lesevorgänge der bestimmten Sequenzen bestimmt wird.

9. Verfahren nach einem der Ansprüche 4 bis 8, wobei die Antigenspezifität einer bestimmten sortierten Einzelzelle in Kombination mit Einzelzellen-TCR-Sequenzierung bestimmt wird.

10. Verfahren nach einem der vorhergehenden Ansprüche, wobei die Affinität und das Bindungsmotiv eines bestimmten TCR bestimmt wird.

11. Verfahren nach einem der vorhergehenden Ansprüche, wobei das mindestens eine Nukleinsäuremolekül
a. eine Länge von 20-200 Nukleotiden, wie etwa 20-150, wie etwa 20-100, wie etwa 30-100, wie etwa 30-80, wie etwa 30-50 Nukleotiden aufweist, und/oder
b. DNA, RNA und/oder künstliche Nukleotide wie etwa PNA oder LNA umfasst oder daraus besteht, wie etwa DNA oder RNA umfasst oder daraus besteht; und/oder
c. einen zentrale Strecke von Nukleinsäuren (Barcoderegion), die von 10 bis mehr als 50 Nukleotiden reicht, umfasst.

12. Verfahren nach einem der vorhergehenden Ansprüche, wobei das Grundmolekül ferner eine oder mehrere auswählbare Markierungen umfasst, wie etwa eine oder mehrere Markierungen ausgewählt aus der Gruppe bestehend aus Fluoreszenzmarkierungen, His-Tags und Metallionen-Tags.

13. Verfahren nach einem der vorhergehenden Ansprüche, wobei das Grundmolekül aus der Gruppe bestehend aus Polysacchariden, einschließlich Glucanen wie etwa Dextran, einem Streptavidin und einem Streptamer-Multimer ausgewählt ist.

14. Verfahren nach einem der vorhergehenden Ansprüche, wobei der multimere MHC aus mindestens vier bestimmten MHC-Peptidmolekülen besteht, wie etwa mindestens acht, wie etwa mindestens zehn, wie etwa 2-30, wie etwa 2-20, wie etwa 2-10, wie etwa 4-10 oder wie etwa 15-30 bestimmten MHC-Peptidmolekülen.

15. Verfahren nach einem der vorhergehenden Ansprüche, wobei der MHC aus der Gruppe bestehend aus einem MHC der Klasse I, einem MHC der Klasse II, einer CD1 und anderen MHC-ähnlichen Molekülen ausgewählt ist.

16. Verfahren nach einem der vorhergehenden Ansprüche, wobei das Verfahren dem Nachweisen mehrerer verschiedener antigenreaktiver Zellen in einer Probe, wie etwa in einer einzigen Probe, dient.

17. Verfahren nach einem der vorhergehenden Ansprüche, wobei die Probe aus der Gruppe bestehend aus einer Blutprobe, wie etwa einer Probe peripheren Blutes; einer von Blut stammenden Probe; einer Gewebeprobe und einer Körperflüssigkeit, wie etwa Rückenmarksflüssigkeit oder Speichel ausgewählt ist.

## Revendications

1. Procédé de détection de cellules T sensibles à l'antigène dans un échantillon comprenant :
i) la fourniture d'une composition comprenant différents sous-ensembles de CMH multimères, dans lequel chaque CMH multimère comprend i) deux molécules de peptide CMH données ou plus liées à une molécule de squelette, et ii) au moins une molécule d'acide nucléique liée à ladite molécule de squelette, dans lequel ladite au moins une molécule d'acide nucléique comprend une première région d'amorce en 5', un segment central d'acides nucléiques (région de code-barres) et une seconde région d'amorce en 3', dans lequel ladite région de code-barres sert de marqueur spécifique pour lesdites deux molécules de peptide CMH données ou plus ;
dans lequel chaque sous-ensemble de CMH multimères présente une région de code-barres unique spécifique de la molécule de peptide CMH donnée ; et
ii) la mise en contact desdits CMH multimères avec ledit échantillon ; et
iii) l'élimination des CMH multimères non liés avant amplification par lavage et centrifugation ; et
iv) la détection de la liaison des CMH multimères auxdits cellules T sensibles à l'antigène, permettant ainsi de détecter des cellules T sensibles à un antigène présents dans lesdits CMH multimères,
dans lequel ladite liaison desdits CMH multimères auxdits cellules T sensibles à l'antigène est détectée par
a) amplification par PCR de la région de code-barres desdites molécules d'acide nucléique liées aux CMH multimères liés aux cellules, et
b) séquençage desdites régions de code-barres amplifiées.

2. Procédé selon la revendication 1, dans lequel les régions d'amorces desdites molécules d'acide nucléique sont identiques pour chaque sous-ensemble de CMH multimères, et dans lequel dans l'étape iv) a) les régions de code-barres desdites molécules d'acide nucléique sont amplifiées à l'aide d'un ensemble d'amorces.

3. Procédé selon l'une quelconque des revendications 1 et 2, dans lequel ledit procédé comprend en outre le tri d'une ou de plusieurs populations de cellules T sensibles à l'antigène avant amplification.

4. Procédé selon l'une quelconque des revendications 1 à 3, dans lequel ledit procédé comprend en outre un tri mono-cellululaire avant amplification.

5. Procédé selon l'une quelconque des revendications 3 et 4, dans lequel ledit tri cellulaire comprend un procédé choisi dans le groupe constitué de la cytométrie de flux telle que FACS ; de la sélection par billes magnétiques ; de l'exclusion par taille ; de la centrifugation par gradient ; de la fixation sur colonne et de la filtration sur gel.

6. Procédé selon l'une quelconque des revendications précédentes, dans lequel ledit séquençage est choisi parmi le séquençage profond, le séquençage haut débit et le séquençage de nouvelle génération.

7. Procédé selon l'une quelconque des revendications précédentes, dans lequel ladite détection de liaison de cellules sensibles à l'antigène comporte la comparaison de valeurs mesurées à un niveau de référence, tel qu'un témoin négatif et/ou un niveau total de réponse.

8. Procédé selon l'une quelconque des revendications précédentes, dans lequel
a. la fréquence spécifique des cellules T est calculée en comparant la fréquence des lectures de séquences de code-barres au nombre de cellules T triés, ou
b. la contribution relative de multiples complexes de peptide CMH différents liés à un seul cellule T est déterminée par le nombre de lectures de code-barres des séquences données.

9. Procédé selon l'une quelconque des revendications 4 à 8, dans lequel la spécificité à l'antigène d'une cellule unique triée donnée est déterminée en combinaison avec un séquençage par TCR d'une cellule unique.

10. Procédé selon l'une quelconque des revendications précédentes, dans lequel l'affinité et le motif de liaison d'un TCR donné sont déterminés.

11. Procédé selon l'une quelconque des revendications précédentes, dans lequel l'au moins une molécule d'acide nucléique
a. présente une longueur de 20 à 200 nucléotides, telle que de 20 à 150, telle que de 20 à 100, telle que de 30 à 100, telle que de 30 à 80, telle que de 30 à 50 nucléotides, et/ou
b. comprend ou est constituée d'ADN, d'ARN, et/ou de nucléotides artificiels tels que le PNA ou le LNA, par exemple comprend ou est constituée d'ADN ou d'ARN ; et/ou
c. comprend un segment central d'acides nucléiques (région de code-barres) allant de 10 à plus de 50 nucléotides.

12. Procédé selon l'une quelconque des revendications précédentes, dans lequel le squelette comprend en outre un ou plusieurs marqueurs sélectionnables, par exemple un ou plusieurs marqueurs choisis dans le groupe constitué de marqueurs fluorescents, de marqueurs His et de marqueurs à ions métalliques.

13. Procédé selon l'une quelconque des revendications précédentes, dans lequel la molécule de squelette est choisie dans le groupe constitué des polysaccharides, y compris des glucanes tels que le dextrane, d'une streptavidine et d'un multimère de streptamère.

14. Procédé selon l'une quelconque des revendications précédentes, dans lequel ledit CMH multimère est composé d'au moins quatre molécules de peptide CMH données, telle qu'au moins huit, telle qu'au moins dix, telle que de 2 à 30, telle que de 2 à 20, telle que de 2 à 10, telle que de 4 à 10 ou telle que de 15 à 30 molécules de peptide CMH données.

15. Procédé selon l'une quelconque des revendications précédentes, dans lequel le CMH est choisi dans le groupe constitué d'un CMH de classe I, d'un CMH de classe II, d'un CD1, et d'autres molécules de type CMH.

16. Procédé selon l'une quelconque des revendications précédentes, dans lequel ledit procédé est destiné à détecter de multiples cellules différentes sensibles à l'antigène dans un échantillon, tel que dans un échantillon unique.

17. Procédé selon l'une quelconque des revendications précédentes, dans lequel l'échantillon est choisi dans le groupe constitué d'un échantillon de sang, tel qu'un échantillon de sang périphérique ; d'un échantillon dérivé du sang ; d'un échantillon de tissu et un fluide corporel, tel qu'un fluide rachidien ou de la salive.
